(19) Europäisches Patentamt

European Patent Office

Office européen des brevets

(11) **EP 4 238 711 A1**

(12) # EUROPEAN PATENT APPLICATION
published in accordance with Art. 153(4) EPC

(43) Date of publication:
**06.09.2023 Bulletin 2023/36**

(21) Application number: **22791935.4**

(22) Date of filing: **15.04.2022**

(51) International Patent Classification (IPC):
**B25J 9/00** (2006.01)  **B25J 19/02** (2006.01)
**B25J 13/08** (2006.01)  **G01L 1/14** (2006.01)

(52) Cooperative Patent Classification (CPC):
**A61B 5/1071; A61B 5/1114; A61B 5/224;
A61B 5/6812;** A61B 2503/08

(86) International application number:
**PCT/KR2022/005110**

(87) International publication number:
**WO 2022/225235 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.04.2021 KR 20210050468
27.10.2021 KR 20210144559**

(71) Applicant: **Samsung Electronics Co., Ltd.
Suwon-si, Gyeonggi-do 16677 (KR)**

(72) Inventors:
• **HWANG, Jungsik**
  **Suwon-si Gyeonggi-do 16677 (KR)**
• **KIM, Kyungrock**
  **Suwon-si Gyeonggi-do 16677 (KR)**
• **ROH, Changhyun**
  **Suwon-si Gyeonggi-do 16677 (KR)**
• **LEE, Jusuk**
  **Suwon-si Gyeonggi-do 16677 (KR)**
• **HYUNG, Seungyong**
  **Suwon-si Gyeonggi-do 16677 (KR)**

(74) Representative: **Gulde & Partner
Patent- und Rechtsanwaltskanzlei mbB
Wallstraße 58/59
10179 Berlin (DE)**

(54) **METHOD AND DEVICE FOR MEASURING MUSCULAR FITNESS OF USER BY USING
WEARABLE DEVICE**

(57)    A method of measuring muscular fitness of a
user using a wearable device includes determining a target resistance profile for a target movement to be performed by the user wearing the wearable device to measure muscular fitness, controlling a motor driver circuit of
the wearable device based on the target resistance profile to control a resistance force to be provided to the
user, measuring state information of an actual movement
performed by the user under the resistance force, and
measuring the muscular fitness of the user based on the
state information.

FIG. 6

EP 4 238 711 A1

**Description**

BACKGROUND

**1. Field**

Methods and apparatuses consistent with example embodiments relate to a method and device for measuring muscular fitness of a user, and more particularly, a method and device for measuring muscular fitness of a user wearing a wearable device.

**2. Description of the Related Art**

Aging demographics have contributed to a growing number of people who experience inconvenience and pain from reduced muscular strength or joint problems due to aging. Thus, there is a growing interest in walking assist devices that enable elderly users or patients with reduced muscular strength or joint problems to walk with less effort.

SUMMARY

One or more example embodiments may address at least the above problems and/or disadvantages and other disadvantages not described above. Also, the example embodiments are not required to overcome the disadvantages described above, and an example embodiment may not overcome any of the problems described above.

According to an aspect of various example embodiments, there is provided a method of measuring muscular fitness performed by a wearable device, the method including determining a target resistance profile for a target movement to be performed by a user wearing the wearable device, controlling a motor driver circuit of the wearable device based on the target resistance profile to control a motor of the wearable device in providing a resistance force to the user, measuring or obtaining state information of a movement performed by the user under the resistance force, and measuring or determining muscular fitness of the user based on the state information.

According to another aspect of various example embodiments, there is provided a wearable device including a memory in which a program for measuring muscular fitness of a user is recorded, a processor configured to execute the program, a communication module configured to exchange data with an external device, a sensor configured to measure a first angle of a first joint of the user, a motor driver circuit configured to be controlled by the processor, and a motor electrically connected to the motor driver circuit. The program may be configured to perform operations of determining a target resistance profile for a target movement to be performed by the user wearing the wearable device, controlling the motor driver circuit of the wearable device based on the target resistance profile to control the motor in providing a resistance force to the user, measuring or obtaining state information of a movement performed by the user under the resistance fore, and measuring or determining muscular fitness of the user based on the state information.

According to yet another example embodiment, there is provided an exoskeleton for measuring muscular fitness of a limb of a user, including: a first support member configured to be put on a first limb part of the limb of a user; a second support member configured to be put on a second limb part of the limb of the user; a driver and sensor, the driver and the sensor being connected to at least one of the first and the second support member; a memory storing a program for measuring muscular fitness; a processor configured to execute the program, the program being configured to: determining a target resistance profile for a target movement to be performed by the user wearing the exoskeleton on the limb; control the driver to apply the target resistance profile; obtaining information regarding the movement of the first and the second support members; determining the muscular fitness of the first limb part and the second limb parts of the user, wherein the muscular fitness relates to at least one of peak torque, work, muscular power, muscular endurance, torque acceleration energy, acceleration time, and a range of motion.

BRIEF DESCRIPTION OF THE DRAWINGS

FIGS. 1A through 1D are diagrams illustrating a wearable device according to an example embodiment.
FIG. 2 is a diagram illustrating a wearable device communicating with an electronic device according to an example embodiment.
FIGS. 3 and 4 are diagrams illustrating an example of outputting a torque by a wearable device according to an example embodiment.
FIG. 5 is a diagram illustrating a user measuring muscular fitness through a wearable device according to an example embodiment.

FIG. 6 is a flowchart illustrating a method of measuring muscular fitness of a user according to an example embodiment.

FIG. 7 is a flowchart illustrating a method of activating a muscular fitness measurement mode according to an example embodiment.

FIG. 8 is a flowchart illustrating a method of transmitting target movement guide information to a user terminal according to an example embodiment.

FIG. 9 is a diagram illustrating a guide video output to a user terminal according to an example embodiment.

FIG. 10 is a diagram illustrating examples of a resistance profile according to an example embodiment.

FIG. 11 is a flowchart illustrating a method of controlling a motor driver circuit based on a resistance profile according to an example embodiment.

FIGS. 12 through 15 are diagrams illustrating a motor driver circuit of a wearable device according to an example embodiment.

FIG. 16 is a diagram illustrating a screen output to a user terminal during measurement of muscular fitness according to an example embodiment.

FIG. 17 is a flowchart illustrating a method of calculating muscular fitness based on a plurality of indicators according to an example embodiment.

FIG. 18 is a flowchart illustrating a method of calculating a plurality of indicators according to an example embodiment.

FIG. 19 is a diagram illustrating a sensed joint angle and a torque calculated based on the joint angle according to an example embodiment.

FIG. 20 is a flowchart illustrating a method of providing an assistance force to a user when an operation mode is an assistance mode according to an example embodiment.

FIG. 21 is a flowchart illustrating a method of calculating an assistance torque value based on muscular fitness of a user according to an example embodiment.

FIGS. 22A through 22C are diagrams illustrating a full body-type wearable device according to another example embodiment.

FIG. 23 is a flowchart illustrating a method of measuring muscular fitness performed by a user terminal according to an example embodiment.

DETAILED DESCRIPTION

Reference will now be made in detail to example embodiments, examples of which are illustrated in the accompanying drawings, wherein like reference numerals refer to the like elements throughout.

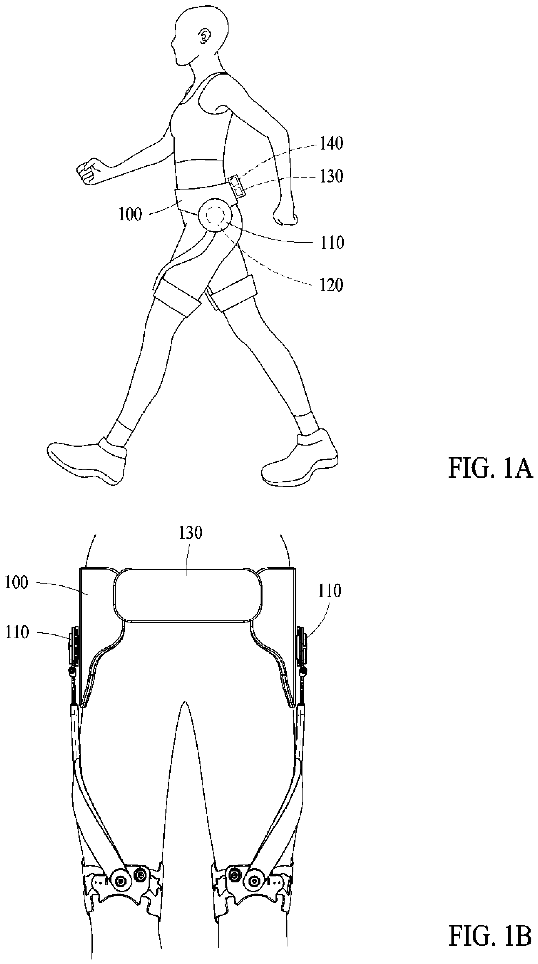

FIGS. 1A through 1D are diagrams illustrating a wearable device according to an example embodiment.

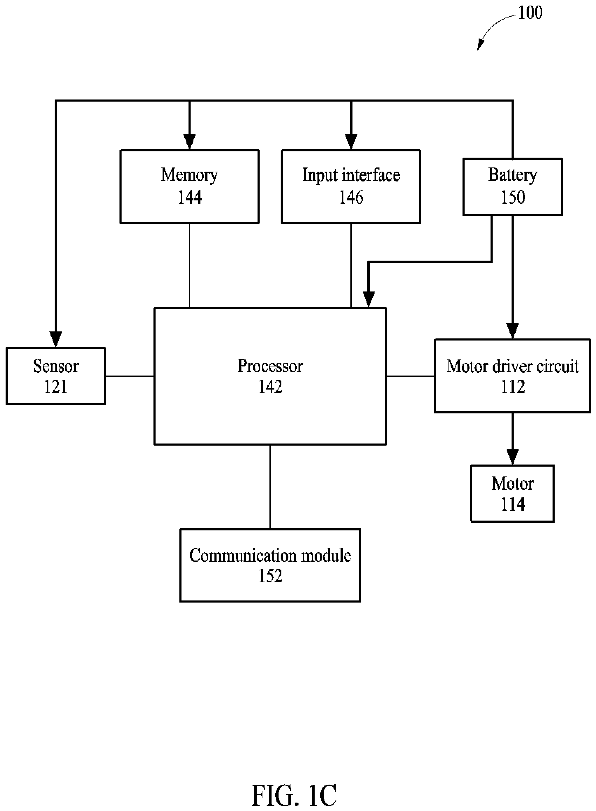

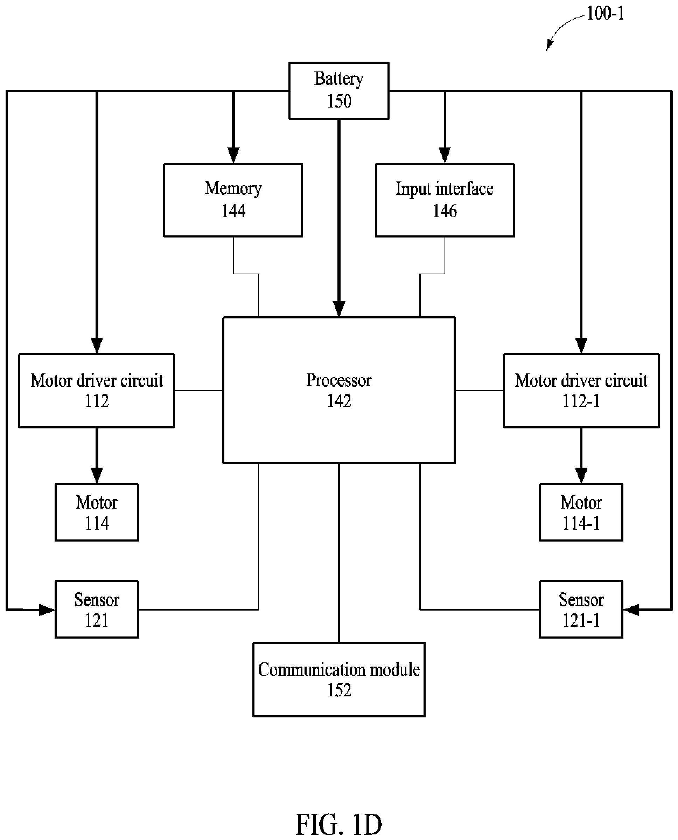

Referring to FIG. 1, a wearable device 100 may be worn on a user and configured to assist the user in walking more readily. The wearable device 100 may be a device that assists the user in walking. The wearable device 100 may also be an exercise device that provides the user with a resistance force to assist the user in doing an exercise. The resistance force provided to the user may be, for example, a force output by a device such as a motor, but a force that hinders a movement of the user, a force that acts in an opposite direction of a direction in which the user or a body part of the user moves, etc. The resistance force may also be referred to as an exercise load.

Although FIGS. 1A through 1D illustrate an example of a hip-type wearable device, a type of the wearable device 100 is not limited to the illustrated hip type, and the wearable device 100 may be provided in a type that supports a whole lower body, supports a portion of the lower body, for example, a portion of the lower body up to a knee and a portion of the lower body up to an ankle, or supports a whole body.

Although example embodiments to be described hereinafter with reference to FIGS. 1A through 1D are applicable to a hip-type wearable device, for example, the wearable device 100, the example embodiments are not limited to the hip-type wearable device, but applicable to all types of wearable devices

Referring to FIGS. 1A through 1D, the wearable device 100 may include a driver 110, a sensor 120, an inertial measurement unit (IMU) 130, a controller 140, a battery 150, and a communication module 152. For example, the IMU 130 and the controller 140 may be arranged in a main frame of the wearable device 100. For another example, the IMU 130 and the controller 140 may be included in a housing formed in or attached to the outside of the main frame of the wearable device 100.

The driver 110 may include a motor 114 and a motor driver circuit 112 for driving the motor 114. The sensor 120 may include at least one sensor 121. The controller 140 may include a processor 142, a memory 144, and an input interface 146. Although the sensor 121, the motor driver circuit 112, and the motor 114 are illustrated in FIG. 1C as a single sensor, a single motor driver circuit, and a single motor, respectively, a wearable device 100-1 according to another example embodiment may include a plurality of sensors 121 and 121-1, a plurality of motor circuits 112 and 112-1, and a plurality of motors 114 and 114-1 as illustrated in FIG. 1D. According to implementation, the wearable device 100 may include a plurality of processors. The number of motor driver circuits, the number of motors, or the number of processors may vary according to a body part on which the wearable device 100 is worn.

The following descriptions of the sensor 121, the motor driver circuit 112, and the motor 114 may also be applicable to the sensor 121-1, the motor driver circuit 112-1, and the motor 114-1 illustrated in FIG. 1D.

The driver 110 may drive a hip joint of the user. For example, the driver 110 may be disposed at o near a right hip of the user and/or at or near a left hip of the user. The driver 110 may be additionally disposed at or near knees of the user and at or near ankles of the user. The driver 110 may include the motor 114 configured to generate a rotational torque and the motor driver circuit 112 configured to drive the motor 114.

The sensor 120 may measure an angle of the hip joint (hereinafter also be referred to as a hip joint angle) of the user when the user walks. Here, information associated with the hip joint angle sensed by the sensor 120 may include a right hip joint angle, a left hip joint angle, a difference between the right hip joint angle and the left hip joint angle, and a hip joint motion direction. For example, the sensor 121 may be disposed in the driver 110. Based on a position of the sensor 121, the sensor 120 may additionally measure a knee angle of the user and an ankle angle of the user. The sensor 121 may be an encoder. The information associated with the hip joint angle measured by the sensor 120 may be transmitted to the controller 140.

According to an example embodiment, the sensor 120 may include a potentiometer. The potentiometer may sense an R-axis joint angle and an L-axis joint angle, and an R-axis joint angular velocity and an L-axis joint angular velocity, based on a walking motion of the user. In this case, R and L axes may be reference axes for a right leg and a left leg of the user, respectively. For example, the R and L axes may be set to be vertical to the ground and set such that a front side of a body of a person has a negative value and a rear side of the body has a positive value.

The IMU 130 may measure acceleration information and pose information when the user walks. For example, the IMU 130 may sense an X-axis acceleration, a Y-axis acceleration, and a Z-axis acceleration, and an X-axis angular velocity, a Y-axis angular velocity, and a Z-axis angular velocity, based on a walking motion of the user. The acceleration information and the pose information measured by the IMU 130 may be transmitted to the controller 140.

In addition to the sensor 120 and the IMU 130 described above, the wearable device 100 may include other sensors (e.g., an electromyogram (EMG) sensor) configured to sense a change in a quantity of motion of the user or a change in biosignal based on a walking motion of the user.

The controller 140 may control an overall operation of the wearable device 100. For example, the controller 140 may receive information sensed by each of the sensor 120 and the IMU 130. The information sensed by the IMU 130 may include the acceleration information and the pose information, and the information sensed by the sensor 120 may include information on the right hip joint angle, the left hip joint angle, the difference between the angles of the two hip joints, and the hip joint motion direction. According to example embodiments, the controller 140 may calculate the difference between the angles of both hip joints based on the right hip joint angle and the left hip joint angle. The controller 140 may generate a signal for controlling the driver 110 based on the sensed information. For example, the generated signal may be an assistance force for assisting the user in walking. For another example, the generated signal may be a resistance force for preventing the user from walking. The resistance force may be provided to the user to assist the user in doing an exercise or motion.

According to an example embodiment, the processor 142 of the controller 140 may control the driver 110 to provide the resistance force to the user.

For example, the driver 110 may provide the resistance force to the user using back-drivability of the motor 114 without outputting a torque to the user. Here, the back-drivability of a motor may represent reactivity of a rotation axis of the motor in response to an external force, and a greater back-drivability may indicate that the motor may more readily respond to an external force acting on the rotation axis, that is, the rotation axis of the motor may more readily rotate. For example, even when the same external force is applied to the rotation axis of the motor, a degree of rotation of the rotation axis of the motor may change according to a degree of the back- drivability.

For another example, the driver 110 may provide the resistance force to the user by outputting a torque in a direction that hinders the user from moving or that provides resistance to the user.

The user may apply, to the wearable device 100, a force against the resistance force provided to the user to measure muscular fitness. The muscular fitness may be calculated using measurement indicators, such as, for example, a peak torque (PT, unit: J/rad), work (W, unit: W), muscular power (MP, unit: W), and muscular endurance (ME, unit: repetition (rep)), torque acceleration energy (TAE, unit: J), acceleration time (AT, unit: sec), and a range of motion (RoM, unit: deg). The muscular power refers to a force a muscle produces in a possible shortest period of time, and is different from muscular strength which refers to a force a muscle produces regardless of time.

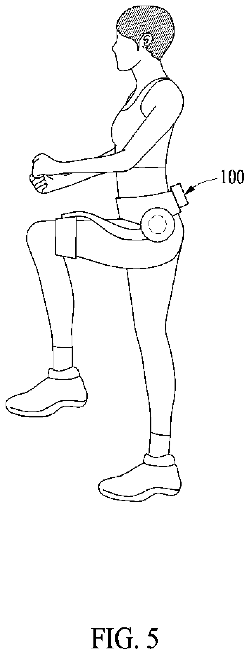

The wearable device 100 may calculate the preset indicators by quantifying an applied force. The muscular fitness of the user may be measured based on these indicators. A method of measuring muscular fitness of a user will be described in detail with reference to FIGS. 5 through 21.

According to another example embodiment, the processor 142 of the controller 140 may control the driver 110 such that the driver 110 outputs a torque (or an assistance torque) for assisting the user in walking. For example, in the hip-type wearable device 100, the driver 110 may be disposed at or near each of a left hip and a right hip of the user, and the controller 140 may output a control signal for controlling the driver 110 to generate a torque.

The driver 110 may generate the torque based on the control signal output by the controller 140. A torque value for generating the torque may be externally set or be set by the controller 140. For example, to indicate a magnitude of the torque value, the controller 140 may use a magnitude of a current for a signal transmitted to the driver 110. That is, as the magnitude of the current received by the driver 110 increases, the torque value may increase. For another example, the processor 142 of the controller 140 may transmit the control signal to the motor driver circuit 112 of the driver 110, and the motor driver circuit 112 may generate a current corresponding to the control signal to control the motor 114.

The battery 150 may supply power to the components of the wearable device 100. The wearable device 100 may further include a circuit (e.g., a power management integrated circuit (PMIC)) configured to convert power of the battery 150 to match an operating voltage of the components of the wearable device 100 and provide it to the components of the wearable device 100. In addition, the battery 150 may or may not supply power to the motor 114 based on an operation mode of the wearable device 100. That is, the battery 150 may supply power to the motor 114 through the motor driver circuit 112 in an assistance mode, and may not supply power to the motor 114 in a muscular fitness measurement mode. Accordingly, the battery 150 may consume less power in the muscular fitness measurement mode, which may increase the use time (or service time) of the wearable device 100.

The communication module 152 may support the establishment of a direct (or wired) communication channel or a wireless communication channel between the wearable electronic device 100 and an external electronic device, and support the communication through the established communication channel. The communication module 152 may include one or more communication processors that support direct (or wired) communication or wireless communication. The communication module 152 may include, for example, a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with an external electronic device through a first network (e.g., a short-range communication network such as Bluetooth, wireless-fidelity (Wi-Fi) direct, or infrared data association (IrDA)) or a second network (e.g., a legacy cellular network, a 5G network, a next-generation communication network, the Internet, or a computer network). These types of communication modules may be integrated into a single component (e.g., a single chip) or different separate components (e.g., chips).

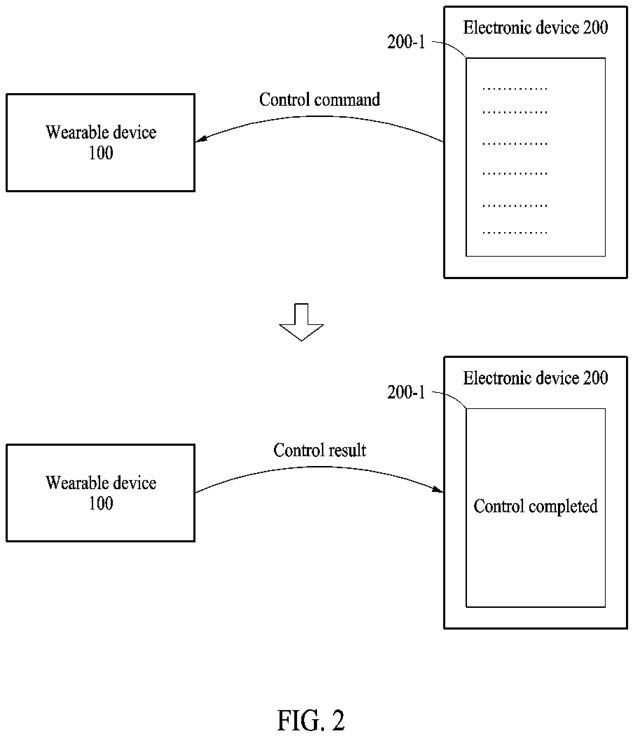

FIG. 2 is a diagram illustrating a wearable device communicating with an electronic device according to an example embodiment.

Referring to FIG. 2, the wearable device 100 may communicate with an electronic device 200. The electronic device 200 may include, for example, a smartphone, a tablet personal computer (PC), a smartwatch, eyeglasses, and the like, but is not limited to the foregoing example. For example, the electronic device 200 may be an electronic device of a user of the wearable device 100. For another example, the electronic device 200 may be an electronic device of a trainer who guides the user wearing the wearable device 100.

According to implementation, the wearable device 100 and the electronic device 200 may communicate via a server (not shown) through short-range wireless communication or cellular mobile communication.

The electronic device 200 may display a user interface (UI) for controlling an operation of the wearable device 100 on a display 200-1. The UI may include, for example, at least one soft key through which the user may control the wearable device 100.

The user may input a command for controlling the operation of the wearable device 100 through the UI on the display 200-1 of the electronic device 200, and the electronic device 200 may generate a control command corresponding to the command and transmit the generated control command to the wearable device 100. The wearable device 100 may operate according to the received control command and transmit a control result to the electronic device 200. The electronic device 200 may display a control completion message on the display 200-1 of the electronic device 200.

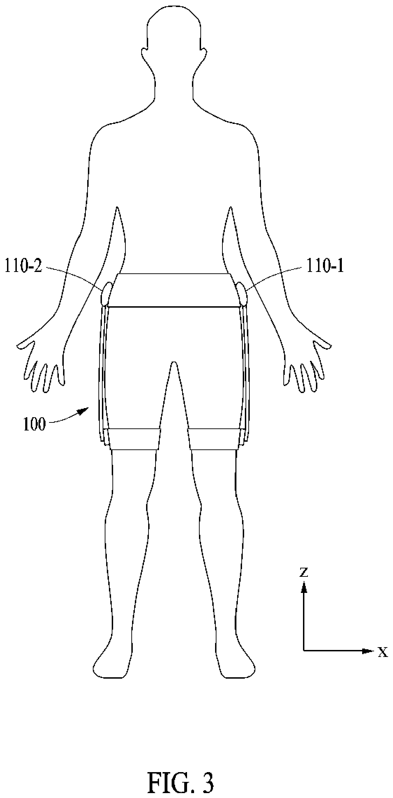

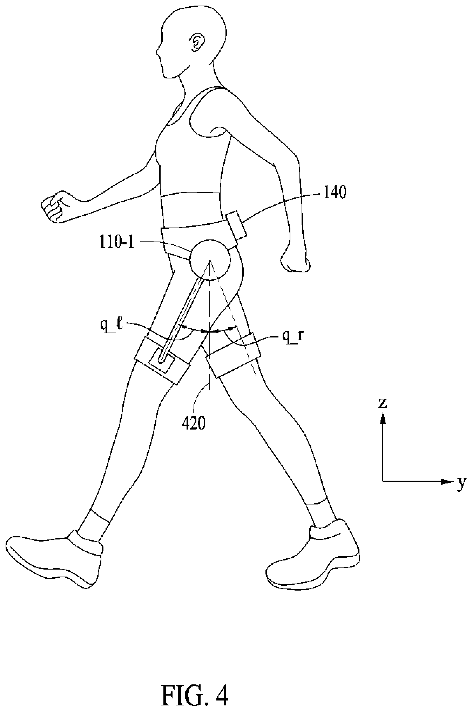

FIGS. 3 and 4 are diagrams illustrating an example of outputting a torque by a wearable device according to an example embodiment;

Referring to FIGS. 3 and 4, the drivers 110-1 and 110-2 of the wearable device 100 of FIGS. 1A through 1D may be disposed at or near a hip joint of a user, and the controller 140 of the wearable device 100 may be disposed at or near a waist of the user. However, the positions of the drivers 110-1 and 110-2 and the controller 140 are not limited to the example positions illustrated in FIGS. 3 and 4.

The wearable device 100 may measure (or sense) a left hip joint angle q_l and a right hip joint angle q_r of the user. For example, the wearable device 100 may measure the left hip joint angle q_l of the user through a left encoder as the sensor 121 and measure the right hip joint angle q_r of the user through a right encoder as the sensor 121-1. As illustrated in FIG. 4, the left hip angle q_l may be a negative value because a left leg of the user is in front of a reference line 420, and the right hip angle q_r may be a positive value because a right leg of the user is behind the reference line 420. According to implementation, the right hip joint angle q_r may be negative when the right leg is in front of the reference line 420, and the left hip joint angle q_l may be positive when the left leg is behind the reference line 420.

According to an example embodiment, the wearable device 100 may determine a torque value $\tau(t)$ based on the left hip joint angle q_l, the right hip joint angle q_r, a gain $\kappa$, and a delay $\Delta t$, and control the motor driver circuit 112 of the wearable device 100 such that the determined torque value $\tau(t)$ is output. A force provided to the user by the torque value $\tau(t)$ may be referred to herein as force feedback. For example, the wearable device 100 may determine the torque value $\tau(t)$ based on Equation 1 below.

[Equation 1]

$$y = sin(q\_r) - sin(q\_l)$$

$$\tau(t) = \kappa y(t - \Delta t)$$

In Equation 1 above, y dentoes a state factor, and q_r denotes a right hip joint angle, and q_l denotes a left hip joint angle. According to Equation 1, the state factor y may indicate a distance between both legs. For example, y being 0 may indicate a state (e.g., a crossing state) in which the distance between the legs is 0, and an absolute value of y being maximum may indicate a state (e.g., a landing state) in which an angle between the legs is maximal. When q_r and q_l are measured at a time t, the state factor may be represented as y(t).

The gain $\kappa$ is a parameter indicating a magnitude and direction of an output torque. As the magnitude of the gain $\kappa$ increases, a greater torque may be output. When the gain $\kappa$ is a negative value, a torque acting as a resistance force may be output to the user. When the gain $\kappa$ is a positive value, a torque acting as an assistance force may be output to the user. The delay $\Delta t$ is a parameter associated with a torque output timing. The gain $\kappa$ and the delay $\Delta t$ may be preset, and be adjusted by the user or the wearable device 100.

FIG. 5 is a diagram illustrating a user measuring muscular fitness through a wearable device according to an example embodiment.

According to an example embodiment, a user may measure muscular fitness with the wearable device 100 on. The wearable device 100 may provide a resistance force to the user to measure the muscular fitness. For example, the resistance force may be provided based on back-drivability of the motor 114. For another example, the resistance force may be provided based on a frictional force by a gear ratio of the wearable device 100. For still another example, the resistance force may be provided based on a torque generated by the motor 114.

The wearable device 100 may measure a movement of the user against the resistance force provided to the user through a sensor and measure the muscular fitness based on a value measured by the sensor. The movement of the user is illustrated as knee lifting in FIG. 5. The wearable device 100 may quantify the movement of the user and calculate the muscular fitness based on a numerical value obtained by quantifying the movement.

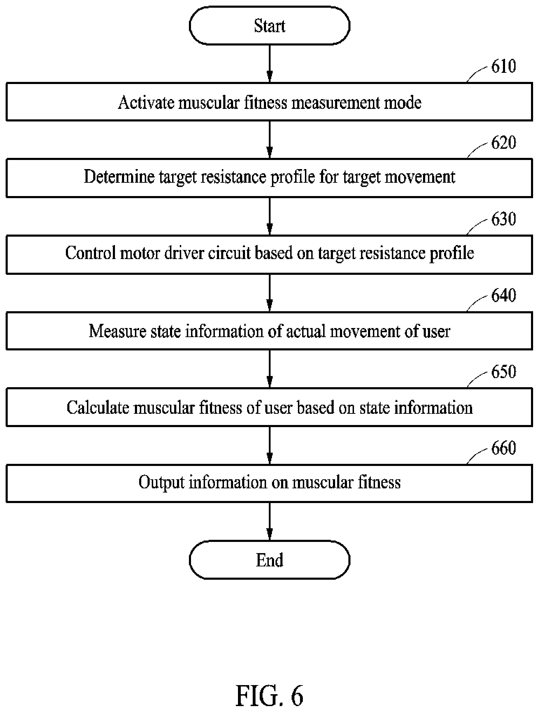

FIG. 6 is a flowchart illustrating a method of measuring muscular fitness of a user according to an example embodiment.

According to an example embodiment, operations (e.g., operations 610 through 660) to be described hereinafter with reference to FIG. 6 may be performed by the controller 140 (or the processor 142 of the controller 140) of the wearable device 100.

In operation 610, the processor 142 may activate a muscular fitness measurement mode. For example, when receiving an input that selects the muscular fitness measurement mode from a user, the processor 142 may activate the muscular fitness measurement mode.

In operation 620, the processor 142 may determine a target resistance profile for a target movement that is one or more movements to be performed by the user to measure the muscular fitness. For example, the target resistance profile for the target movement may be determined from among a plurality of previously generated resistance profiles.

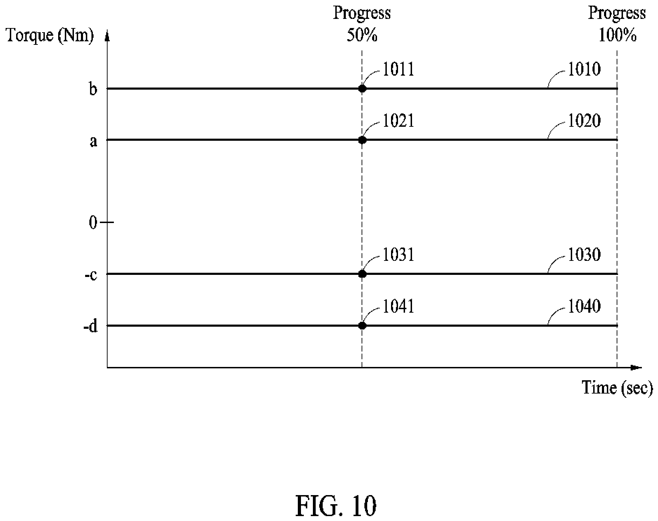

According to an example embodiment, a previously generated resistance profile may indicate a trajectory of a resistance force for an entire muscular fitness measurement cycle. A resistance force corresponding to a current progress level of the muscular fitness measurement cycle may be determined based on the resistance profile for the entire muscular fitness measurement cycle. The muscular fitness measurement cycle may be represented as a numerical value obtained by quantifying a series of target movements. When a target movement is performed repeatedly, the muscular fitness measurement cycle may also proceed repeatedly. An example of the resistance profile will be described in detail with reference to FIG. 10.

According to an example embodiment, the processor 142 may determine a target movement for measuring muscular fitness from among one or more target movements. For example, a different target movement may be determined according to a progress of the muscular fitness measurement mode.

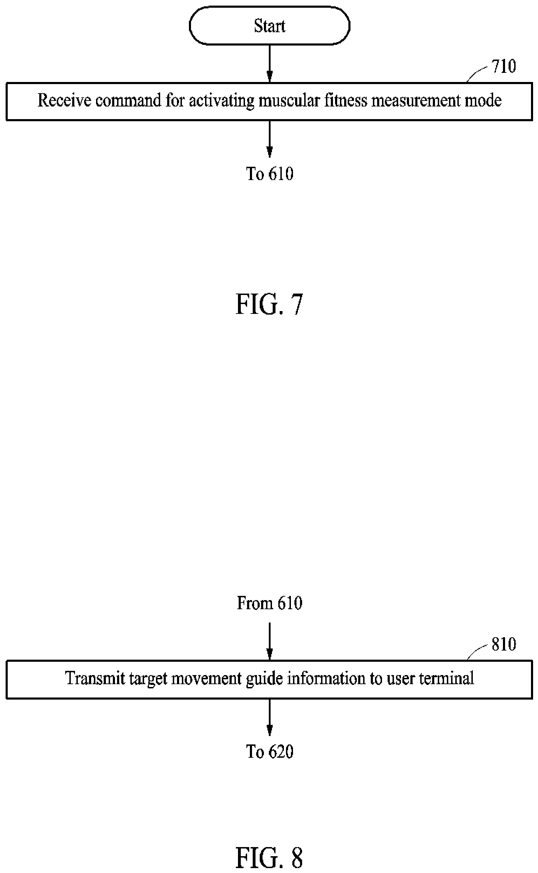

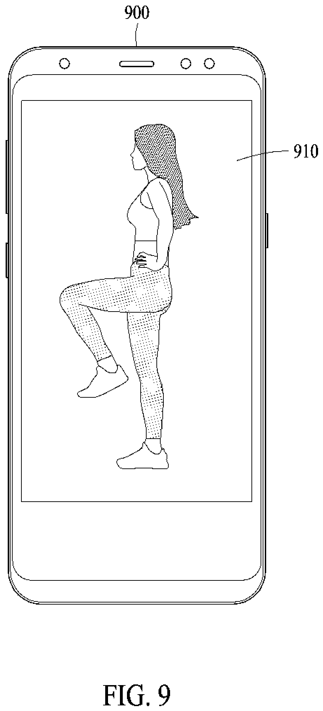

According to an example embodiment, information on the determined target movement may be provided to the user. The providing of the information on the target movement to the user will be described in detail with reference to FIGS. 8 and 9.

A first target movement among the target movements may be knee lifting. Knee lifting. Knee lifting may be a pose that starts from an upright standing pose of the user with both feet in contact with the ground and returns to the standing pose after maximally raising a leg without bending a waist of the user. A second target movement among the target movements may be stretching a leg behind. Stretching a leg behind may be a pose that starts from an upright standing pose of the user with both hands against a wall and returns to the standing pose after maximally raising the leg backward without bending the waist of the user.

The processor 142 may determine the target resistance profile from among the resistance profiles based on a resistance level (e.g., a first level, a second level, a third level, or a fourth level) received from a user terminal. For example, as the received resistance level is higher, a resistance profile having a higher resistance force may be determined.

For example, when the same resistance force is to be provided to the user during measurement of the muscular fitness, a resistance profile having the same resistance force throughout the entire muscular fitness measurement cycle may be determined. For another example, when a resistance force that varies according to a muscular fitness measurement cycle is to be provided, a resistance profile in which the resistance force changes according to a progress level of the muscular fitness measurement cycle may be determined. The progress level of the muscular fitness measurement cycle may be determined based on a time or a joint angle (e.g., a joint angle set) of the user.

In operation 630, the processor 142 may control the motor driver circuit 112 based on the target resistance profile to control the resistance force to be provided to the user.

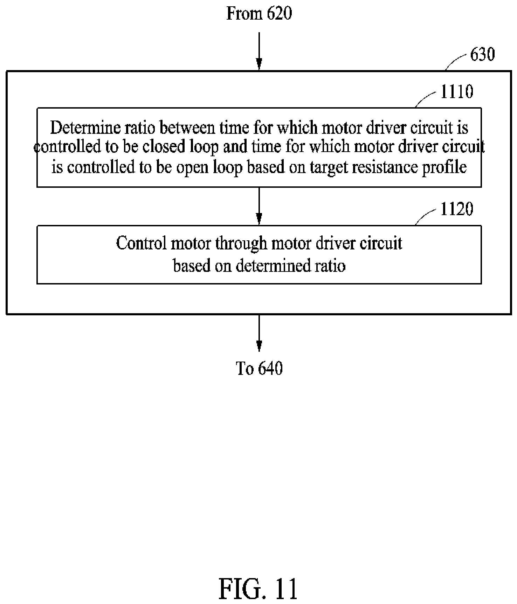

According to an example embodiment, the processor 142 may determine a ratio between a time for which the motor driver circuit 112 is controlled to be a closed loop and a time for which the motor driver circuit 112 is controlled to be an open loop based on the target resistance profile, and control the motor driver circuit 112 based on the determined ratio. The resistance force to be provided to the user may vary based on the determined ratio. The controlling of the motor driver circuit 112 will be described in detail with reference to FIG. 11.

For example, the target resistance profile may have a corresponding execution time, and the processor 142 may control the motor driver circuit 112 during the execution time. The execution time may be controlled through a timer preset for the target movement.

In operation 640, the processor 142 may measure, through the sensor 120 and/or the IMU 130, state information of a movement of the user performed under the resistance force provided by the wearable device 100.

The state information may include at least one of one or more joint torques, joint angles, joint angular velocities, and joint angular acceleration values measured by the sensor 120. The joint angles may include a first angle of a first joint (e.g., a hip joint, a knee joint, or an ankle joint).

For example, when the target movement is knee lifting, the wearable device 100 may determine, on its side, whether the movement is performed based on a measured hip joint angle.

According to an example embodiment, when knee lifting is performed once, the wearable device 100 may provide feedback to the user. For example, the wearable device 100 may provide auditory feedback to the user through a speaker (not shown) of the wearable device 100. For another example, the wearable device 100 may provide auditory feedback to the user through a speaker of the user terminal. For still another example, the wearable device 100 may provide visual feedback to the user through a display of the user terminal. For yet another example, the wearable device 100 may provide tactile feedback to the user through a vibrator (not shown) of the wearable device 100.

To measure muscular fitness, the user may perform knee lifting repeatedly until the timer of the target resistance profile expires.

For another example, when the target movement is stretching a leg behind, the wearable device 100 may determine, on its side, whether the movement is performed based on a measured hip joint angle. When stretching a leg behind is performed once, the wearable device 100 may provide auditory feedback to the user. To measure muscular fitness, the user may perform stretching a leg behind repeatedly until the timer of the target resistance profile expires.

According to another example embodiment, the state information may include at least one of acceleration information and pose information measured by the IMU 130.

According to an example embodiment, while the timer for the execution time of the target resistance profile is running, the state information of the movement of the user may be measured by the sensor 120 and/or the IMU 130. When the timer expires, the measurement of the state information may also be suspended.

In operation 650, the processor 142 may calculate the muscular fitness of the user based on the state information. For example, the processor 142 may numerically calculate a plurality of preset indicators based on the state information, and calculate the muscular fitness based on the calculated indicators.

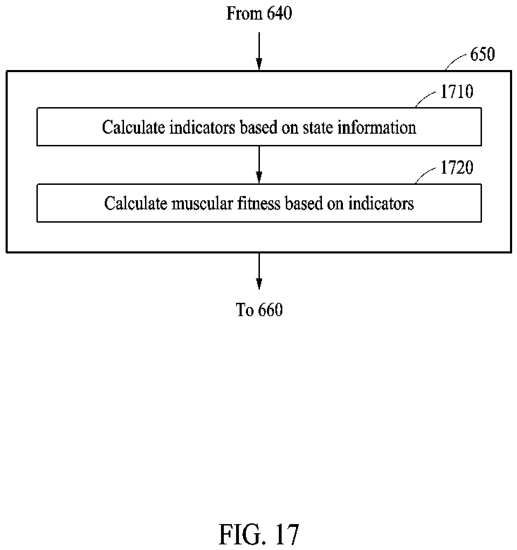

The muscular fitness may be represented as scores using a predefined equation. The calculating of the muscular fitness will be described in detail with reference to FIGS. 17 through 19.

In operation 660, the processor 142 may output information on the muscular fitness. For example, the processor 142 may transmit the information on the muscular fitness to the user terminal through the communication module 152. For another example, the processor 142 may output the information on the muscular fitness through a display (not shown) of the wearable device 100. The user may verify the information on the muscular fitness output on the display of the user terminal or the wearable device 100.

FIG. 7 is a flowchart illustrating a method of activating a muscular fitness measurement mode according to an example embodiment.

According to an example embodiment, operation 710 to be described hereinafter with reference to FIG. 7 may be performed before operation 610 described above with reference to FIG. 6 is performed.

In operation 710, the processor 142 may receive a command for activating a muscular fitness measurement mode from a user.

According to an example embodiment, the user may transmit the command for activating the muscular fitness measurement mode to the wearable device 100 through a user terminal (e.g., the electronic device 200 of FIG. 2) connected to the wearable device 100 through a wireless network. The wearable device 100 may receive the muscular fitness measurement mode through the communication module 152. The wireless network may include, for example, a cellular network, a Bluetooth network, a Wi-Fi network, and the like, but is not limited to the foregoing example.

According to another example embodiment, the user may input the command for activating the muscular fitness measurement mode through the input interface 146 of the wearable device 100. For example, the input interface 146 may include a physical button for receiving an input from the user and a software button formed based on a touch panel, and include a display (not shown) and indicator (not shown) for outputting a state of the wearable device 100. The indicator may be, for example, a light-emitting diode (LED), but is not limited to the foregoing example.

For example, a plurality of operation modes that control the wearable device 100 may be pre-stored in the form of a program in the wearable device 100.

According to an example embodiment, an application for measuring muscular fitness or an application for controlling the wearable device 100 may be pre-installed (or stored) in the user terminal, and the user terminal may operate based on the application.

When the command for activating the muscular fitness measurement mode is received from the user terminal in operation 610, the processor 142 may activate the muscular fitness measurement mode.

FIG. 8 is a flowchart illustrating a method of transmitting target movement guide information to a user terminal according to an example embodiment.

According to an example embodiment, operation 810 to be described hereinafter with reference to FIG. 8 may be performed before operation 620 described above with reference to FIG. 6 is performed after operation 610 described above with reference to FIG. 6 is performed.

In operation 810, the processor 142 may transmit target movement guide information to a user terminal. The user terminal may output a guide video from the user terminal based on the guide information. For example, the guide information may be a guide video or information representing the guide video. An example of the guide video will be described in detail with reference to FIG. 9.

According to an example embodiment, the guide video may be about one or more movements (e.g., a protocol for measuring muscular fitness with target movements) to be performed by a user to measure muscular fitness. The user may understand such a measurement protocol through the guide video and perform (or follow) the movements. The user terminal may output a stored guide video or stream a guide video stored in a server.

The user who views the guide video may learn in advance the movements for measuring muscular fitness.

FIG. 9 is a diagram illustrating a guide video output to a user terminal according to an example embodiment.

Referring to FIG. 9, a user terminal 900 (e.g., the electronic device 200 of FIG. 2) may control the wearable device 100. For example, the user terminal 900 may be a terminal dedicated to controlling the wearable device 100. For another example, the user terminal 900 may be a terminal in which an application for controlling the wearable device 100 is installed.

When a user intends to measure muscular fitness through the wearable device 100, the user terminal 900 may output, through a display, a guide image 910 based on guide information.

According to an example embodiment, when the user inputs a command for activating a muscular fitness measurement mode through the user terminal 900, a preset guide image may be output. For example, when the muscular fitness measurement mode requires a specific movement, a guide image for guiding the movement may be output. The user may verify in advance the movement to be performed during measurement of muscular fitness, through the guide image.

FIG. 10 is a diagram illustrating examples of a resistance profile according to an example embodiment.

According to an example embodiment, a resistance profile may represent a trajectory of a resistance force with respect to an entire muscular fitness measurement cycle.

For example, as illustrated, one or more resistance profiles 1010, 1020, 1030, and 1040 may be preset according to a target movement and a resistance level. For example, the resistance profiles 1010 and 1020 may be set for a first movement. The resistance profiles 1010 and 1020 may output different torques according to the resistance level. The resistance profiles 1030 and 1040 may be set for a second movement. A positive torque value and a negative torque value may indicate opposite torque directions.

Although it is illustrated that a plurality of profiles is set according to the resistance level for the same movement, one profile may be set for a movement.

The illustrated resistance profiles 1010, 1020, 1030, and 1040 may be example resistance profiles applied when the same resistance force is provided to the user during measurement of muscular fitness according to an example embodiment. However, a resistance profile is not limited to the illustrated examples. For example, a resistance profile in which a resistance force changes based on a level of a muscular fitness measurement cycle may be preset.

According to an example embodiment, the processor 142 may determine a resistance force, for example, a resistance force 1011, 1021, 1031, or 1041, corresponding to a current progress level (e.g., 50%) of the muscular fitness measurement cycle, and control the motor driver circuit 112 and the motor 114 to provide the resistance force to the user.

FIG. 11 is a flowchart illustrating a method of controlling a motor driver circuit based on a resistance profile according to an example embodiment.

According to an example embodiment, operation 630 described above with reference to FIG. 6 may include operations 1110 and 1120 to be described hereinafter with reference to FIG. 11.

In operation 1110, the processor 142 may determine a ratio between a time for which the motor driver circuit 112 is controlled to be a closed loop and a time for which the motor driver circuit 112 is controlled to be an open loop, based on a target resistance profile. The ratio between the time for which the motor driver circuit 112 is controlled to be the closed loop and the time for which the motor driver circuit 112 is controlled to be the open loop may also be referred to as a connection ratio. For example, the connection ratio of the motor driver circuit 112 corresponding to a resistance level may be determined. The connection ratio of the motor driver circuit 112 may refer to a ratio by which the motor driver circuit 112 is controlled to be the closed loop (e.g., a first control state) or the open loop (e.g., a second control state) under the condition where energy is controlled not to be provided to the motor 114 from the battery 150. For example, that a circuit connection ratio is 0.5 based on the closed loop may indicate that a state of the closed loop and a state of the open loop are controlled to be 50% and 50%, respectively, within a preset period of time. The circuit connection ratio may be dynamically adjusted in a case where a resistance force changes.

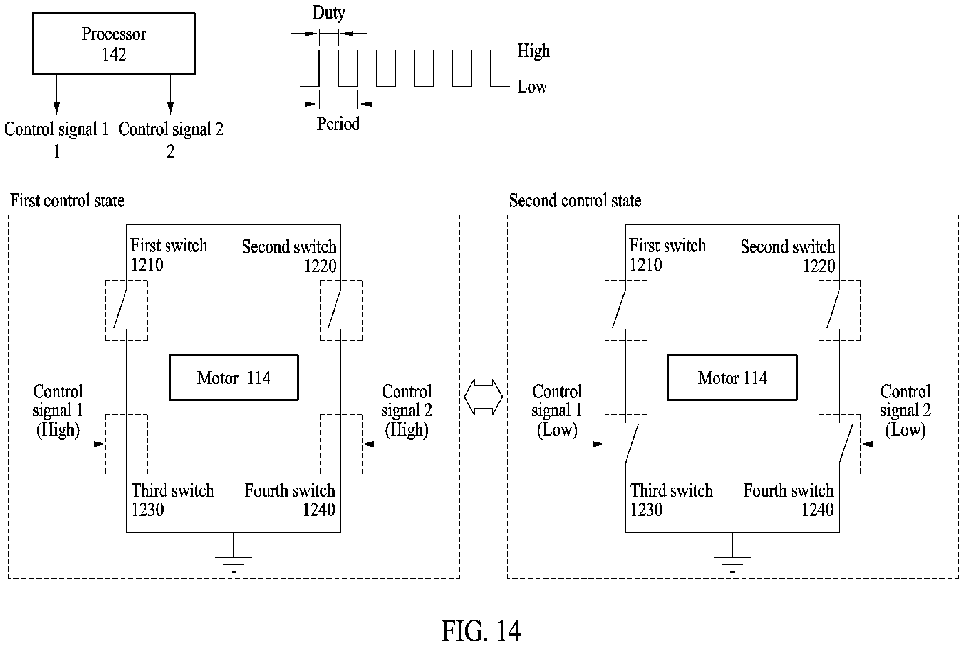

For example, the connection ratio may be implemented using pulse-width modulation (PWM), but is not limited thereto. Various methods of adjusting a connection state of the motor driver circuit 112 may be employed. When PWM is used to control the connection ratio, the connection ratio determined in operation 1110 may be represented as PWM having a specific duty ratio. The PWM having a specific duty ratio may be preset such that a desired resistance force is provided to the user by the PWM. The duty ratio will be described in detail with reference to FIG. 14.

In operation 1120, the processor 142 may control the motor 114 through the motor driver circuit 112 based on the determined ratio.

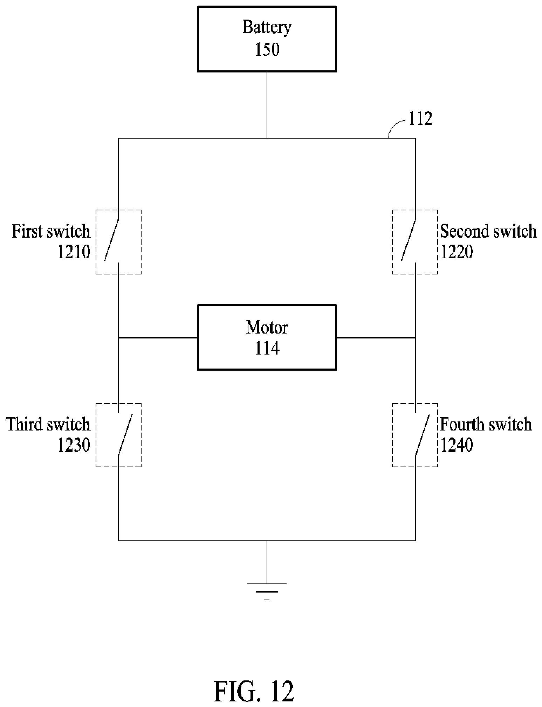

According to an example embodiment, the motor driver circuit 112 may be controlled to be the closed loop (or the first control state) or the open loop (or the second control state) by the PWM having the duty ratio. For example, the processor 142 may control the motor driver circuit 112 to be the closed loop or the open loop by controlling operations of switches in the motor driver circuit 112 using the PWM. Hereinafter, controlling the motor driver circuit 112 to be the closed loop or the open loop by controlling the operations of the switches in the motor driver circuit 112 will be described in detail with reference to FIGS. 12 through 15.

When the motor driver circuit 112 is the closed loop, back-drivability of the motor 114 may decrease by dynamic braking. As the back-drivability decreases, a resistance force experienced by the user may increase. In contrast, when the motor driver circuit 112 is the open loop, the back-drivability of the motor 114 may increase, and only a frictional force by a gear ratio may be experienced (or felt) by the user as a resistance force.

A desired back-drivability may be obtained by adjusting such an open-closed state ratio of the motor driver circuit 112 based on the connection ratio. For example, the resistance force provided to the user may be adjusted by the ratio by which the motor driver circuit 112 is controlled to be the closed loop or the open loop within a uniform and repetitive period of time of the PWM. When the ratio of the time for which the motor driver circuit 112 is controlled to be the closed loop increases, the resistance force may increase.

FIGS. 12 through 15 are diagrams illustrating a motor driver circuit of a wearable device according to an example embodiment.

The motor driver circuit 112 illustrated in FIG. 12 may be an H-bridge circuit and include a plurality of switches 1210 through 1240. The motor driver circuit 112 may be connected to the motor 114.

When a first switch 1210 and a fourth switch 1240 are closed and a second switch 1220 and a third switch 1230 are open under the control of the processor 142, a closed loop including the battery 150 may be formed, and thus power may be supplied from the battery 150 to the motor 114. The motor 114 may rotate in a first direction.

Conversely, when the second switch 1220 and the third switch 1230 are closed and the first switch 1210 and the fourth switch 1240 are open under the control of the processor 142, a closed loop including the battery 150 may also be formed, and thus power may be supplied from the battery 150 to the motor 114. In this case, however, the motor 114 may rotate in a second direction which is opposite to the first direction.

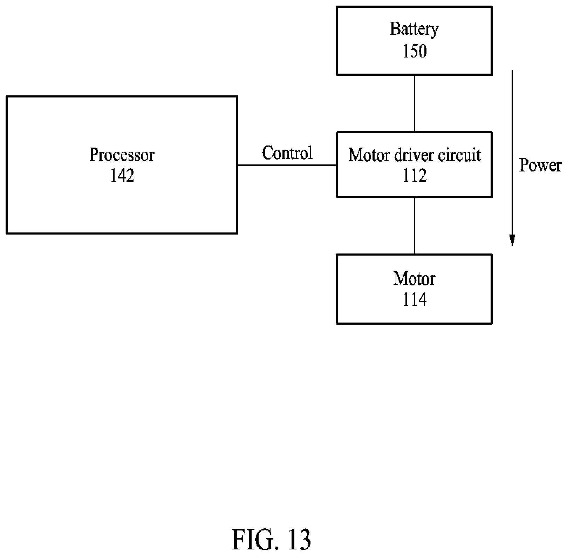

FIG. 13 is a diagram illustrating an example case in which the motor driver circuit 112 is controlled to form a closed loop including the battery 150 and the battery 150 supplies power to the motor 114. The motor 114 may rotate in a direction corresponding to a current direction, as described above with reference to FIG. 12. For example, when an operation mode of the wearable device 100 is an assistance mode, the processor 142 may control the motor driver circuit 112 to form the closed loop including the battery 150. For another example, even when the operation mode of the wearable device 100 is a muscular fitness measurement mode, for example, when a torque needs to be output through the motor 114, the processor 142 may control the motor driver circuit 112 to form the closed loop including the battery 150.

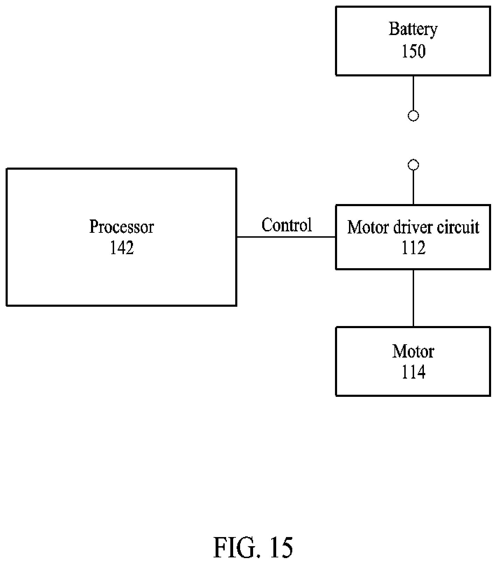

Unlike the example case where the motor driver circuit 112 is controlled to form the closed loop including the battery 150 as described above with reference to FIGS. 12 and 13, an example case in which the motor driver circuit 112 is controlled not to use the battery 150 will be described hereinafter with reference to FIGS. 14 and 15. A method of controlling the motor driver circuit 112 to be described hereinafter with reference to FIGS. 14 and 15 may also be used when the operation mode of the wearable device 100 is the muscular fitness measurement mode.

Referring to FIG. 14, the processor 142 may disconnect an electrical connection between the battery 150 and the motor 114 by opening the first switch 1210 and the second switch 1220. In the muscular fitness measurement mode, the first switch 1210 and the second switch 1220 may be maintained in an open state. In the muscular fitness measurement mode, only a lower driver circuit including the third switch 1230 and the fourth switch 1240 connected to the motor 114 may be controlled.

The processor 142 may apply control signal 1 to the third switch 1230 and apply control signal 2 to the fourth switch 1240 such that a control state of the motor driver circuit 112 is changed between a first control state and a second control state. Control signals 1 and 2 may be in the form of PWM in which a high value and a low value are alternate repeatedly, and have a duty ratio. The duty ratio may indicate a connection ratio. The duty ratio may be represented as $t_H/T$ when one period is T and a time for which the high value is maintained during one period T is $t_H$. Although outputting separate control signals 1 and 2 by the processor 142 is described above, it is provided merely as an example. For another example, the processor 142 may output a single control signal, and the output control signal may be divided by a separate circuit and the divided control signals may be respectively applied to the third switch 1230 and the fourth switch 1240.

When control signals 1 and 2 are the high value, a positive (+) terminal and a negative (-) terminal of the motor 114 may be connected to each other to form an equipotential state. That is, in the first control state, the + terminal and the - terminal of the motor 114 may be electrically connected to each other to have the same potential (or voltage).

In the first control state, the motor 114 may form a closed loop with the ground without an electrical connection with the battery 150, and thus the first control state may also be referred to as a closed loop state of the motor 114 without the electrical connection with the battery 150.

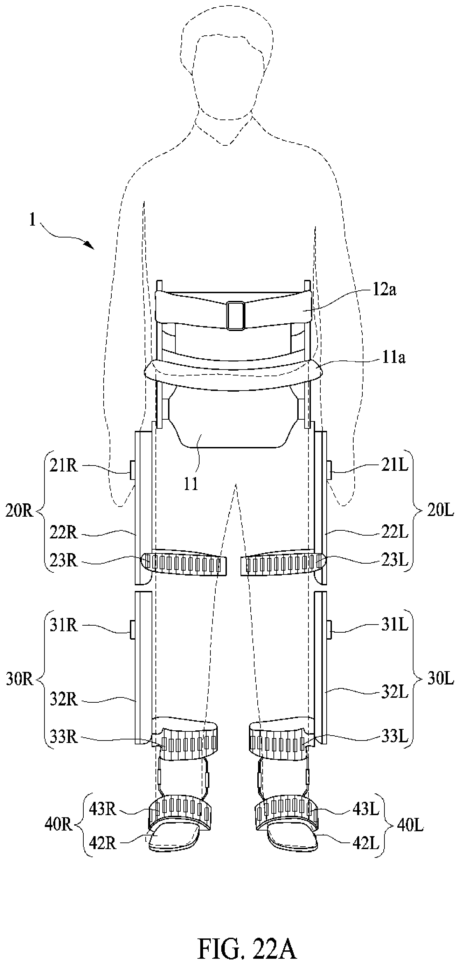

When the user moves in the first control state, the movement of a corresponding joint of the user may allow a device portion (e.g., 20R, 20L, 30R, 30L, 40R, or 40L of FIGS. 22A through 22C) of the wearable device 100 to rotate the motor 114 disposed around the joint, and this rotation may generate an electromotive force (or a potential difference) in the motor 114. In the first control state, the terminals of the motor 114 may be in the equipotential state, and the motor 114 may generate a rotational resistance to reduce the generated electromotive force. This rotational resistance may be provided as a resistance force to the user.

When control signals 1 and 2 are the low value, the + terminal and the - terminal of the motor 114 may be electrically open. Since there is no electrical connection with the motor 114 in the second control state, the second control state may also be referred to as an open loop state of the motor 114.

When the user moves in the second control state, the motor 114 may rotate by the movement of the user. The + terminal and the - terminal of the motor 114 may be electrically open in the second control state, and thus the electromotive force described above may not occur in the motor 114, and the resistance force may not be output. That is, back-drivability of the motor 114 may increase, and only a frictional force by a gear ratio may be experienced or felt by the user as the resistance force.

Since control signals 1 and 2 have the high value and the low value that alternate based on a PWM signal, the control state of the motor driver circuit 112 may be alternately or repeatedly switched between the first control state and the second control state.

The processor 142 may adjust the magnitude of the resistance force by controlling a duty ratio of each of control signals 1 and 2. When a time for which the high value is maintained in a period of each of control signals 1 and 2 increases, that is, when a time for which the low value is maintained decreases, the motor 114 may have a higher ratio of operating in the first control state than operating in the second control state in the period of each of control signals 1 and 2, and thus the intensity of the resistance force output to the user may increase. Conversely, when the time for which the high value is maintained in the period of each of control signals 1 and 2 decreases, that is, when the time for which the low value is maintained in the period of each of control signals 1 and 2 increases, the motor 114 may have a higher ratio of operating in the second control state than operating in the first control state in the period of each of control signals 1 and 2, and thus the intensity of the resistance force output to the user may decrease.

In an exercise mode, the wearable device 100 may output the resistance force without supplying power of the battery 150 to the motor 114, and thus the power of the battery 150 may be less consumed and the use time (or service time) of the wearable device 100 may be improved. In addition, when the power of the battery 150 is supplied to the motor 114, the motor 114 may incorrectly operate. However, the power of the battery 150 may not be supplied to the motor 114 in the muscular fitness measurement mode, and thus the potential incorrect operation of the motor 114 may be prevented and the safety of the wearable device 100 may be further improved.

FIG. 15 illustrates an example case in which the motor driver circuit 112 is controlled not to use the battery 150. The battery 150 may be electrically disconnected from the motor 114. Based on a connection state of the motor driver circuit 112, the motor 114 may generate an electromotive force by an external force (when the connection state corresponds to a closed loop) and may not generate the electromotive force (when the connection state corresponds to an open loop).

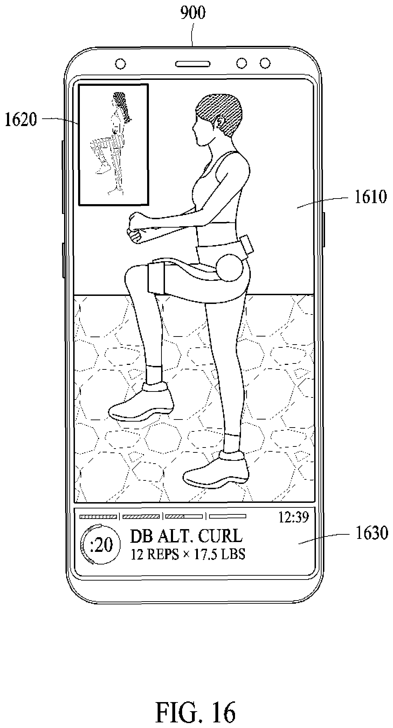

FIG. 16 is a diagram illustrating a screen output to a user terminal during measurement of muscular fitness according to an example embodiment.

According to an example embodiment, while a user is measuring muscular fitness through the wearable device 100, information on a muscular fitness measurement session may be output to the user through the user terminal 900.

For example, the information output to the user terminal 900 may include an image 1610 of the user captured through a camera of the user terminal 900. The user may thus view a pose performed by the user through the image 1610. That is, the pose of the user may be fed back through the image 1610.

For example, the information output to the user terminal 900 may include a guide image 1620. A progress level of the output guide image 1620 may correspond to a progress level of the muscular fitness measurement session. The user may correct the pose of the user by referring to the guide image 1620.

For example, the information output to the user terminal 900 may include information 1630 on the progress level of the muscular fitness measurement session. The processor 142 of the wearable device 100 may transmit a progress level of a target resistance profile (or a muscular fitness measurement cycle) to the user terminal 900 through the communication module 152, and the user terminal 900 may output such received information to the user. For example, the information 1630 on the progress level may include a name of a movement (e.g., DB ALT. CURL, etc.), the number of repetitions of the movement (e.g., 12 REPS), a magnitude of a provided resistance force (e.g., 17.5 LBS), and the like.

FIG. 17 is a flowchart illustrating a method of calculating muscular fitness based on a plurality of indicators according to an example embodiment.

According to an example embodiment, operation 650 described above with reference to FIG. 6 may include operations 1710 and 1720 to be described hereinafter with reference to FIG. 17.

In operation 1710, the processor 142 may calculate a plurality of preset indicators based on state information measured for a movement of a user. The indicators may include at least one of a peak torque (PT), work (W), muscular power (MP), muscular endurance (ME), torque acceleration energy (TAE), an acceleration time (AT), and a range of motion (RoM). Although the peak torque, the work, the muscular power, the muscular endurance, the torque acceleration energy, the acceleration time, and the range of motion are described as examples of the indicators, all of these may not be necessarily calculated to calculate muscular fitness. For example, a combination of any two or more of these may be used to calculate muscular fitness.

The peak torque (PT) may represent a highest torque during a movement, and muscular strength may increase as the peak torque increases.

The work (W) may represent a value (e.g., the size of area) obtained by performing an integration on a torque trajectory, and muscular work may increase as the work increases.

The muscular power (MP) may represent a value of total work compared to an actual contraction time during a measurement time.

The muscular endurance (ME) may represent the number of repetitions performed during a measurement time and may represent, for example, how many times a movement is repeated against a resistance force.

The torque acceleration energy (TAE) may represent maximum work performed during an initial 125 milliseconds (ms) of a torque generation, and quick reaction may increase as the torque acceleration energy increases.

The acceleration time (AT) may represent a time used until the peak torque is reached, and quick reaction may increase as the acceleration time decreases.

The range of motion (RoM) may represent a maximum value of a measured angle, and flexibility may increase as the range of motion increases.

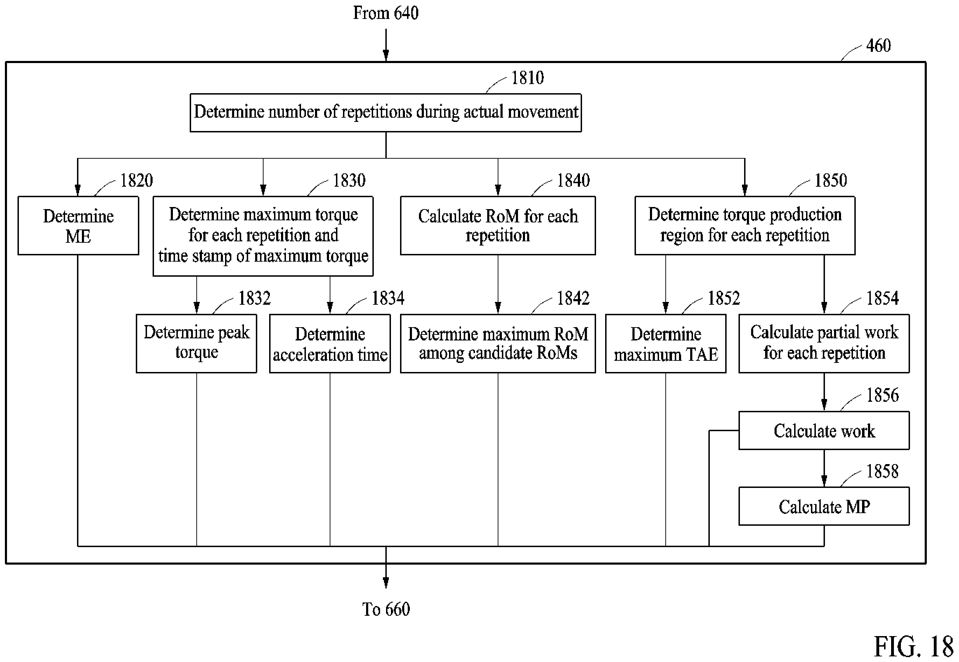

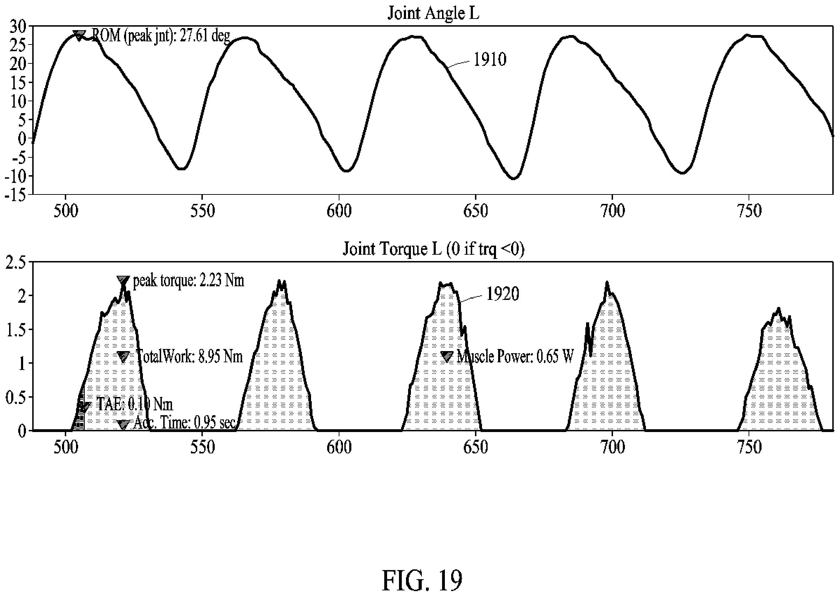

Hereinafter, calculating the indicators will be described in detail with reference to FIGS. 18 and 19.

In operation 1720, the processor 142 may calculate muscular fitness of the user based on the indicators. For example, the muscular fitness may be numerically represented using Equation 2 below.

[Equation 2]

$$Muscle\ Score = a\frac{ResistLevel}{MaxResistLevel} \times bPT \times cW \times dMP \times e\frac{ME}{METhreshold} \times fTAE$$

$$\times g\frac{timeThreshold - AT}{timeThreshold} \times h\frac{ROM}{ROMThreshold}$$

In Equation 2 above, ResistLevel denotes a resistance level, and MaxResistLevel denotes a maximum resistance level. PT denotes a peak torque, W denotes work, MP denotes muscular power, ME denotes muscular endurance, METhreshold denotes a threshold ME value, TAE denotes torque acceleration energy, AT denotes an acceleration time, timeThreshold denotes a threshold AT value, ROM denotes a range of motion, and ROMThreshold denotes a threshold ROM value. In addition, a, b, c, d, e, f, g, and h are constants for the respective indicators.

Hereinafter, calculating muscular fitness of a user based on a plurality of indicators will be described in detail with reference to FIG. 19.

FIG. 18 is a flowchart illustrating a method of calculating a plurality of indicators according to an example embodiment.

According to an example embodiment, operation 650 described above with reference to FIG. 6 may include operations 1810 through 1858 to be described hereinafter with reference to FIG. 18.

In operation 1810, the processor 142 may detect the number of repetitions during a movement based on state information. For example, when the movement is repeated performance of knee lifting, the movement may be divided by the number of repetitions of knee lifting based on a hip joint angle. For example, the movement may be divided into first through Nth knee lifting.

In operation 1820, the processor 142 may determine muscular endurance (ME) based on the detected number of repetitions. For example, the number of repetitions may be determined to be the muscular endurance.

In operation 1830, the processor 142 may determine a maximum torque $\tau_{i,Max}$ for each repetition Repi and a time stamp $t_{j,Max}$ of the maximum torque $\tau_{i,Max}$. For example, a maximum torque for each repetition may be calculated based on state information such as a joint angle and a joint angular velocity measured for each repetition, and a time stamp corresponding to a time at which the maximum torque occurs may be determined to be a time stamp of the maximum torque. Each state information may include a time stamp for a time at which corresponding state information is generated.

In operation 1832, the processor 142 may determine a maximum torque having a greatest value among maximum torques to be a peak torque (PT) $\tau_{Peak}$.

In operation 1834, the processor 142 may determine an acceleration time (AT) $\tau_{Peak}$ for the peak torque $\tau_{Peak}$. The acceleration time may indicate a time used until a peak torque (PT) is reached, and may be determined based on a time stamp of the peak torque.

In operation 1840, the processor 142 may calculate a candidate range of motion RoM$_i$ for each repetition Rep$_i$. For example, a difference between a minimum joint angle JP$_{i,min}$ and a maximum joint angle JP$_{i,Max}$ for each repetition Repi may be calculated to be the candidate range of motion RoM$_i$.

In operation 1842, the processor 142 may determine a maximum range of motion among candidate ranges of motion RoM$_i$.

In operation 1850, the processor 142 may determine a torque production region Region$_i$ for each repetition Rep$_i$. The torque production region Region$_i$ may be calculated by a difference between a start time $t_{i,0}$ of a repetition and a time $t_{i,MaxJP}$ at which the maximum joint angle JP$_{i,Max}$ is reached. That is, the torque production region Region$_i$ may be defined from the start time $t_{i,0}$ of the repetition to the time $t_{i,MaxJP}$ at which the user no longer produces or generates a torque.

In operation 1852, the processor 142 may calculate maximum torque acceleration energy (TAE). For example, the torque acceleration energy may be calculated to be a value obtained by dividing the area of a torque for a preset initial time (e.g., initial 125 ms) of each torque production region Region$_i$ by an angular displacement. The angular displacement may be an angle by which a joint rotates. That is, the torque acceleration energy may be a maximum value among work values W$_{Regioni}$ performed within the initial time (e.g., initial 125 ms) of each torque production region

Region$_i$. The initial 125 ms is provided herein as an example of the torque production region, and thus the initial time may be changed.

In operation 1854, the processor 142 may calculate partial work Wi for each repetition Repi. For example, the partial work Wi may be calculated to be a value obtained by dividing the area of a torque for the torque production region Region$_i$ by the angular displacement.

In operation 1856, the processor 142 may calculate work (W). For example, the work may be calculated to be a sum of partial work values W$_i$.

In operation 1858, the processor 142 may calculate muscular power (MP). For example, the muscular power may be calculated by dividing a work value by an actual contraction time. The actual contraction time may be calculated to be a sum of times for the torque production region Region$_i$.

FIG. 19 is a diagram illustrating a sensed joint angle and a torque calculated based on the joint angle according to an example embodiment.

According to an example embodiment, the wearable device 100 may measure a joint angle as state information. The state information may be generated when the sensor 120 of the wearable device 100 senses a movement of a user under a constant resistance force (e.g., a gain of -4.0) output by the wearable device 100. When the state information is the joint angle, a joint torque trajectory 1920 generated by the user may be calculated based on a joint angle trajectory 1910. The wearable device 100 may calculate a plurality of indicators based on the joint angle trajectory 1910 and the joint torque trajectory 1920. For example, the indicators may be calculated through operations 1810 through 1858 described above with reference to FIG. 18.

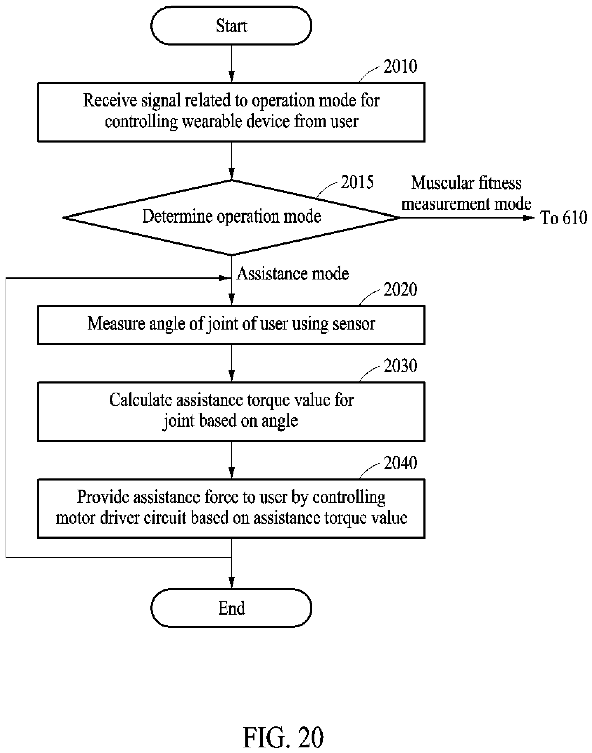

FIG. 20 is a flowchart illustrating a method of providing an assistance force to a user when an operation mode is an assistance mode according to an example embodiment.

According to an example embodiment, operations 2010 through 2040 to be described hereinafter with reference to FIG. 20 may be performed by the wearable device 100. Although the wearable device 100 of FIG. 1 is illustrated as being worn on a lower body of a user, examples are not limited thereto. For example, the wearable device 100 may be worn on an upper body of the user. For another example, the wearable device 100 may be worn around a full body of the user.

In operation 2010, the wearable device 100 may receive a signal associated with an operation mode for controlling the wearable device 100 from a user terminal (e.g., the electronic device 200 of FIG. 2 or the user terminal 900 of FIG. 9), or receive a user input that selects the operation mode. The operation mode may include a muscular fitness measurement mode and an assistance mode, and the received operation mode may be the muscular fitness measurement mode or the assistance mode.

According to an example embodiment, a user may transmit a command (or a signal) for activating a specific operation mode to the wearable device 100 through the user terminal connected to the wearable device 100 through a wireless network. The wearable device 100 may receive the command for activating the operation mode through the communication module 152. The wireless network may include, for example, a cellular network, a Bluetooth network, a Wi-Fi network, and the like, but is not limited to the foregoing example.

According to another example embodiment, the user may select the operation mode through the input interface 146 of the wearable device 100. The input interface 146 may include, for example, a physical button for receiving a user input from the user and a software button formed based on a touch panel, and include a display and indicator outputting a state of the wearable device 100. The indicator may be an LED, but is not limited thereto.

A plurality of dynamic modes for controlling the wearable device 100 may be previously stored in the wearable device 100 in the form of a program.

According to an example embodiment, the processor 142 may control an operation of the wearable device 100 through a control algorithm instead of a model pre-trained based on a neural network. The control algorithm may be an algorithm that uses, as an input value of the algorithm, an input from the user and a value (e.g., state information) generated by the sensor 120 and the IMU 130, and outputs a control value corresponding to the input value. For example, the control algorithm may operate based on a control table representing output values corresponding to input values.

According to another example embodiment, the processor 142 may control the operation of the wearable device 100 based on a model trained in advance based on a neural network corresponding to each operation mode. The neural network may be an artificial neural network, and the neural network for the model may be trained in advance through machine learning. The neural network may be, for example, a convolutional neural network (CNN), a recurrent neural network (RNN), a deep neural network (DNN), or a combination thereof, but is not limited to thereto. The model corresponding to each operation mode may be provided in the form of a program, and be stored in the memory 144 of the wearable device 100.

According to another example embodiment, the processor 142 may control the operation of the wearable device 100 based on a model trained in advance based on a neural network that processes both the assistance mode and the muscular fitness measurement mode.

In operation 2015, the processor 142 may determine whether the operation mode is the muscular fitness measurement mode or the assistance mode. When the operation mode is the muscular fitness measurement mode, a resistance force may be provided to the user. When the operation mode is the assistance mode, an assistance force may be provided to the user. Operations 620 through 660 may be performed to provide the resistance force to the user, and operations 2020 through 2040 may be performed to provide the assistance force to the user.

For example, although operation 2015 is described as being performed to determine the operation mode, operation 2015 may include determining whether the operation mode is the assistance mode and determining whether the operation mode is the muscular fitness measurement mode when the operation mode is not the assistance mode. For another example, operation 2015 may include determining whether the operation mode is the muscular fitness measurement mode and determining whether the operation mode is the assistance mode when the operation mode is not the muscular fitness measurement mode.

In operation 2020, the processor 142 may measure a j oint angle of a j oint of the user using the sensor 121. The sensor 121 may be an angle sensor (e.g., an encoder), and measure an angle of at least one of a hip joint, a knee joint, and an ankle joint. For example, respective angles of a plurality of joints of the user may be measured. The angles of the joints measured simultaneously may constitute a joint angle set with respect to a specific time. The joint angle set may be used to determine a level of a gait cycle of the user. For example, a pose of legs of the user may be determined based on the angles of the hip joint, the knee joint, and the ankle joint. Additionally, an angular acceleration calculated based on a variation in the same joint angle may be included in the joint angle set.

Although the hip joint, the knee joint, and the ankle joint are provided as an example, the foregoing description may also be applicable to other joints (e.g., a shoulder joint, an elbow joint, and a wrist joint) in addition to the described joints of a lower body.

In operation 2030, the processor 142 may calculate an assistance torque value for the joint based on the measured joint angle. For example, the assistance torque value may be determined in response to an input of the joint angle through a control algorithm. For another example, the assistance torque value may be output by inputting the joint angle to a model determined based on the operation mode.

According to an example embodiment, the processor 142 may calculate a state factor which is described above with reference to FIGS. 3 and 4 based on the measured joint angle and determine a gain $\kappa$ and a delay $\Delta t$ with respect to the state factor based on the control algorithm to calculate the assistance torque value for the joint.

According to an example embodiment, the processor 142 may determine a gait state of the user or a level of the gait cycle of the user based on the joint angle, and determine the assistance torque value corresponding to the determined gait state or the determined level of the gait cycle. As the number of joints from which angles are to be measured increases, a more accurate gait state or a more accurate level of a gait cycle may be determined.

The assistance torque value may be determined based on a preset torque profile. Calculating the assistance torque value may not be limited to the foregoing example embodiments.

In operation 2040, the processor 142 may provide an assistance force to the user by controlling the motor 114 based on the assistance torque value. The wearable device 100 may drive the motor 114 using the battery 150 of the wearable device 100 to output an assistance torque, and the assistance force may be provided to the user by the assistance torque output by the motor 114. The assistance torque value may indicate a control signal applied to the motor 114, the assistance torque may indicate a rotational torque output by the motor 114 based on the assistance torque value, and the assistance force may be a force experienced or felt by the user by the assistance torque.

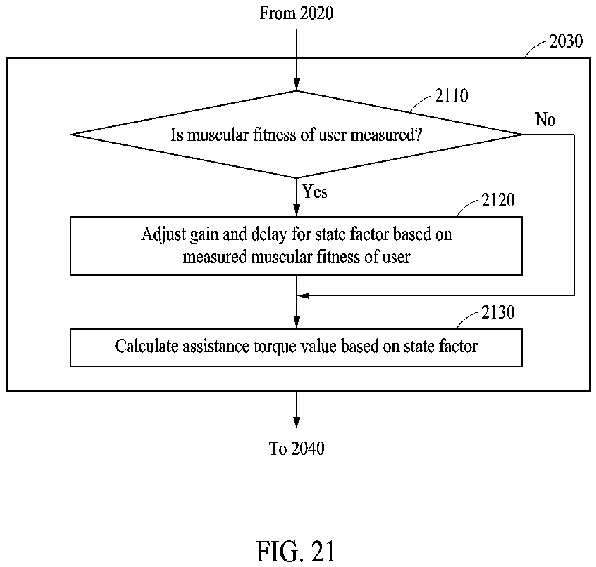

FIG. 21 is a flowchart illustrating a method of calculating an assistance torque value based on muscular fitness of a user according to an example embodiment.

According to an example embodiment, operation 2030 described above with reference to FIG. 20 may include operations 2110 through 2130 to be described hereinafter with reference to FIG. 21.

In operation 2110, the wearable device 100 may determine whether muscular fitness of a user is measured. For example, the wearable device 100 may determine whether there is a record of measurement of the muscular fitness of the user. For another example, through an external device connected to the wearable device 100, the wearable device 100 may determine whether the record of the measurement of the muscular fitness is stored in the external device.

In operation 2120, when the muscular fitness of the user is measured, the wearable device 100 may adjust a gain and a delay for a state factor based on the measured muscular fitness. For example, as the measured muscular fitness increases, the gain may increase while the delay may decrease. For another example, as the measured muscular fitness increases, the gain may decrease. Adjusting the gain and the delay for the state factor based on the muscular fitness may correspond to a personalization of the wearable device 100.

In operation 2130, the wearable device 100 may calculate an assistance torque value based on the state factor calculated based on the measured joint angle.

<Overview of full body-type walking assist device>

FIGS. 22A through 22C are diagrams illustrating a full body-type wearable device according to another example embodiment.

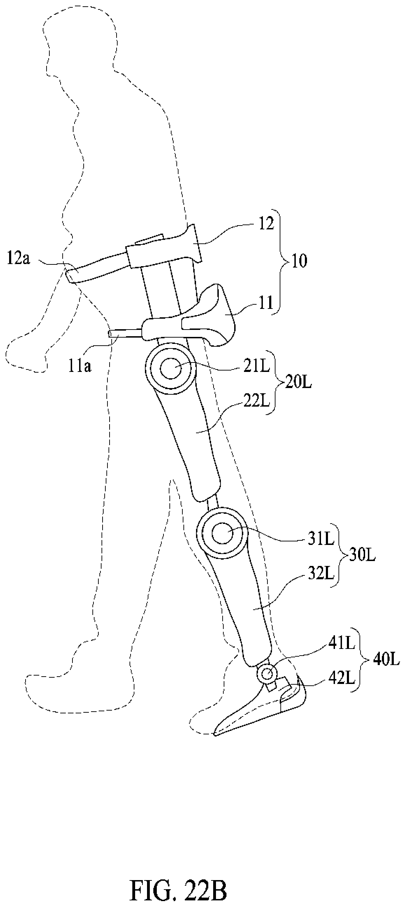

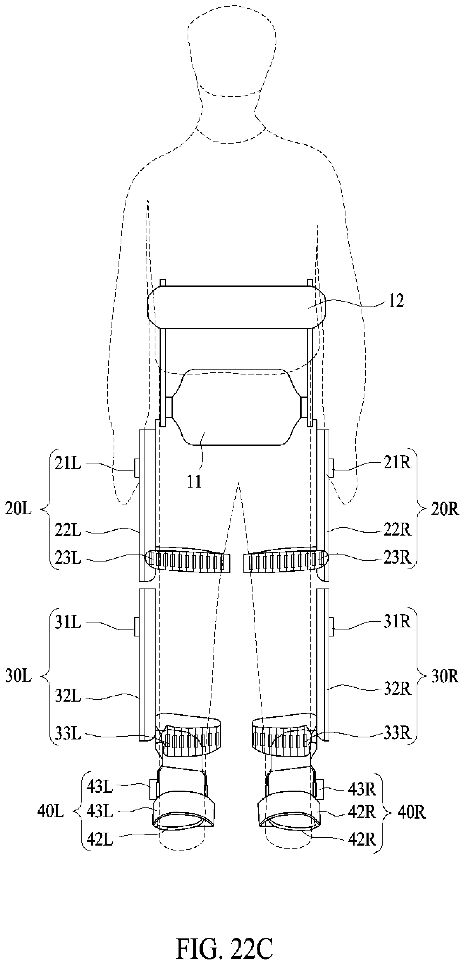

FIG. 22A is a front view of a full body-type wearable device 1, FIG. 22B is a side view of the full body-type wearable device 1, and FIG. 22C is a rear view of the full body-type wearable device 1.

According to an example embodiment, the full body-type wearable device 1 may include a driver 110, a sensor 120, an IMU 130, a controller 140, and a battery 150.

As illustrated in FIGS. 22A through 22C, the full body-type wearable device 1 may be provided in an exoskeleton structure to be worn on a left leg and a right leg of a user. With the wearable device 1 on, the user may be able to perform various movements, for example, extension, flexion, adduction, and abduction. In an embodiment, the exoskeleton structure may be of a full body-type. In another embodiment, the exoskeleton may be of a partial body-type in being worn over one or more limbs, or over one or more parts of the body. The extension may indicate a movement or an exercise of stretching joints, and the flexion may indicate a movement or an exercise of bending joints. The adduction may indicate a movement or an exercise of moving legs to be closer to a central axis of a body, and the abduction may indicate a movement or an exercise of stretching legs in a direction receding from a central axis of a body.

Referring to FIGS. 22A through 22C, the wearable device 1 may include a main body portion 10 and a device portion including right first structure 20R and left first structure 20L, right second structure 30R and left second structure 30L, and right third structure 40R and left third structure 40L.

In an exemplary embodiment, the device portions may include a first support member that is designed to be put on a part of a limb of the user and a second support member that is designed to be put on another part of the limb of the user, so that a joint in the limb is allowed to have natural movement. Further, there is a driver and sensor, the driver and the sensor being connected to at least one of the first support member and the second support member so as to provide resistance against the movement of the limb and to detect certain parameters relating to muscular fitness.

The main body portion 10 may include a housing 11 in which various components are embedded. The components embedded in the housing 11 may include, for example, a central processing unit (CPU), a printed circuit board (PCB), various types of storage devices, and a power source. The main body portion 10 may include the controller 140 described above. The controller 140 may include the CPU and the PCB.

The CPU may be a microprocessor. The microprocessor may be provided with an arithmetic logic operator, a register, a program counter, an instruction decoder, and/or a control circuit in a silicon chip. The CPU may select a control mode that is suitable for a walking environment, and generate a control signal for controlling an operation of the device portion (e.g., first structure 20, second structure 30, and third structure 40).

The PCB may be a board on which a circuit is printed, and the CPU and/or the various storage devices may be installed in the PCB. The PCB may be fixed to an inner side surface of the housing 11.

The various types of storage devices may be embedded in the housing 11. The storage devices may include, for example, a magnetic disk storage device configured to store data by magnetizing a magnetic disk surface, a semiconductor memory device configured to store data using various types of memory semiconductors, and the like.

The power source embedded in the housing 11 may supply power to the various components embedded in the housing 11 or the device portion (e.g., first structure 20, second structure 30, and third structure 40).

The main body portion 10 may further include a waist support 12 configured to support a waist of the user. The waist support 12 may be provided in a shape of a curved flat surface plate that supports the waist of the user.

The main body portion 10 may further include a fixing portion 11a configured to fix the housing 11 to a hip portion of the user, and a fixing portion 12a configured to fix the waist support 12 to the waist of the user. The fixing portions 11a and 12a may be formed with one of a band, a belt, and a strap, all of which are elastic.

The main body portion 10 may include the IMU 130 described above. For example, the IMU 130 may be provided inside or outside the housing 11. The IMU 130 may be provided on the PCB provided inside the housing 11. The IMU 130 may measure an acceleration and an angular velocity

The device portion may include a first structure 20, a second structure 30, and a third structure 40 as illustrated in FIGS. 22A through 22C.

The first structure 20 including a right portion 20R and a left portion 20L may assist or support a movement of a thigh and a hip joint of the user when the user walks. The first structure 20 may include a first driving device including right first driving device 21R and left first driving device 21L, a first support including right first support 22R and left first support 22L, and a first fixing portion including right first fixing portion 23R and left first fixing portion 23L.

The driver 110 described above may include the first driving device including right first driving device 21R and left first driving device 21L. The description of the driver 110 provided above with reference to FIGS. 1 through 16 may be replaced with the description of the first driving device including right first driving device 21R and left first driving device 21L to be provided hereinafter with reference to FIGS. 22A through 22C.

The first driving device including right first driving device 21R and left first driving device 21L may be disposed on the hip joint of the first structure 20 including right first structure 20R and left first structure 20L, and generate a rotational force of different magnitudes in a certain direction. The rotational force generated in the first driving device including right first driving device 21R and left first driving device 21L may be applied to the first support including right first support 22R and left first support 22L. The first driving device including right first driving device 21R and left first driving device 21L may be set to rotate within a motion range of a hip joint of a human body.

The first driving device including right first driving device 21R and left first driving device 21L may operate based on a control signal provided by the main body portion 10. The first driving device including right first driving device 21R and left first driving device 21L may be provided as one of a motor, a vacuum pump, and a hydraulic pump, but is not limited thereto.

A joint angle sensor may be provided around the first driving device including right first driving device 21R and left first driving device 21L. The joint angle sensor may detect an angle by which the first driving device including right first driving device 21R and left first driving device 21L rotates on a rotation axis. The sensor 120 described above may include the joint angle sensor.

The first support including right first support 22R and left first support 22L may be physically connected to the first driving device including right first driving device 21R and left first driving device 21L. The first support including right first support 22R and left first support 22L may rotate in a certain direction by the rotational force generated from the first driving device right first driving device 21R and left first driving device 21L.

The first support including right first support 22R and left first support 22L may be provided in various shapes. For example, the first support including right first support 22R and left first support 22L may be provided in a shape in which nodes are connected with each other. A joint may be between the nodes, by which the first support including right first support 22R and left first support 22L may be bent within a certain range . For another example, the first support including right first support 22R and left first support 22L may be provided in a shape of a rod. In this example, the first support including right first support 22R and left first support 22L may be formed with a flexible material that may be bent within a certain range.

The first fixing portion including right first fixing portion 23R and left first fixing portion 23L may be provided in the first support including right first support 22R and left first support 22L. The first fixing portion including right first fixing portion 23R and left first fixing portion 23L may fix the first support including right first support 22R and left first support 22L to the thigh of the user.

FIGS. 22A through 22C illustrate an example case in which the first support including right first support 22R and left first support 22L is fixed on an outer side of the thigh of the user by the first fixing portion including right first fixing portion 23R and left first fixing portion 23L. When the first driving device including right first driving device 21R and left first driving device 21L operates and the first support including right first support 22R and left first support 22L rotates, the thigh to which the first support including right first support 22R and left first support 22L is fixed may also rotate in the same direction as a rotation direction in which the first support including right first support 22R and left first support 22L rotates.

The first fixing portion including right first fixing portion 23R and left first fixing portion 23L may be formed with one of a band, a belt, and a strap, all of which are elastic, or with a metal material. FIGS. 22A through 22C illustrate, as a chain, the first fixing portion including right first fixing portion 23R and left first fixing portion 23L.

The second structure 30 including a right portion 30R and a left portion 30L may assist or support a movement of a calf and a knee joint of the user when the user walks. The second structure 30 including right second structure 30R and left second structure 30L may include a second driving device including right second driving device 31R and left second driving device 31L, a second support including right second support 32R and left second support 32L, and a second fixing portion including right second fixing portion 33R and left second fixing portion 33L.

The second driving device including right second driving device 31R and left second driving device 31L may be disposed on the knee joint of the second structure 30 including right second structure 30R and left second structure 30L, and generate a rotational force of different magnitudes in a certain direction. The rotational force generated by the second driving device including right second driving device 31R and left second driving device 31L may be applied to the second support including right second support 32R and left second support 32L. The second driving device including right second driving device 31R and left second driving device 31L may be set to rotate within a motion range of a knee joint of a human body.

The driver 110 described above may include the second driving device including right second driving device 31R and left second driving device 31L. Here, what has been described above in relation to the hip joint with reference to FIGS. 1A through 1D may be similarly and substantially applied to the knee joint.

The second driving device including right second driving device 31R and left second driving device 31L may operate based on a control signal provided by the main body portion 10. The second driving device including right second driving device 31R and left second driving device 31L may be provided as one of a motor, a vacuum pump, and a hydraulic pump, but is not limited thereto.

A joint angle sensor may be provided around the second driving device including right second driving device 31R and left second driving device 31L. The joint angle sensor may detect an angle by which the second driving device including right second driving device 31R and left second driving device 31L rotates on a rotation axis. The sensor 120 described above may include the joint angle sensor.

The second support including right second support 32R and left second support 32L may be physically connected to the second driving device including right second driving device 31R and left second driving device 31L. The second support including right second support 32R and left second support 32L may rotate in a direction by the rotational force generated from the second driving device right second driving device 31R and left second driving device 31L.

The second fixing portion including right second fixing portion 33R and left second fixing portion 33L may be provided in the second support including right second support 32R and left second support 32L. The second fixing portion including right second fixing portion 33R and left second fixing portion 33L may fix the second support including right second support 32R and left second support 32L to the calf of the user. FIGS. 22A through 22C illustrate an example case in which the second support including right second support 32R and left second support 32L is fixed on an outer side of the calf of the user by the second fixing portion including right second fixing portion 33R and left second fixing portion 33L. When the second driving device including right second driving device 31R and left second driving device 31L operates and the second support including right second support 32R and left second support 32L rotates, the calf to which the second support including right second support 32R and left second support 32L is fixed may also rotate in the same direction as a rotation direction in which the second support including right second support 32R and left second support 32L rotates.

The second fixing portion including right second fixing portion 33R and left second fixing portion 33L may be formed with one of a band, a belt, and a strap, all of which are elastic, or with a metal material.

The third structure 40 including a right portion 40R and a left portion 40L may assist or support a movement of an ankle joint and related muscles of the user when the user walks. The third structure 40 including right third structure 40R and left third structure 40L may include a third driving device including right third driving device 41R and left third driving device 41L, a foot support including right foot support 42R and left foot support 42L, and a third fixing portion including right third fixing portion 43R and left third fixing portion 43R and 43L.

The driver 110 described above may include the third driving device including right third driving device 41R and left third driving device 41L. Here, what has been described above in relation to the hip joint withe reference to FIGS. 1A through 1D may be similarly and substantially applied to the ankle joint.

The third driving device including right third driving device 41R and left third driving device 41L may be disposed on the ankle joint of the third structure 40 including right third structure 40R and left third structure 40L, and operate based on a control signal provided by the main body portion 10. Similar to the first driving device including right first driving device 21R and left first driving device 21L or the second driving device including right second driving device 31R and left second driving device 31L, the third driving device including right third driving device 41R and left third driving device 41L may be provided as a motor.

The foot support including right foot support 42R and left foot support 42L may be disposed at a position corresponding to a sole of a foot of the user, and physically connected to the third driving device including right third driving device 41R and left third driving device 41L.

The third fixing portion including 43R and 43R may be provided in the foot support including right foot support 42R and left foot support 42L. The third fixing portion including right third fixing portion 43R and left third fixing portion 43R and 43L may fix the foot of the user to the foot support including right foot support 42R and left foot support 42L.

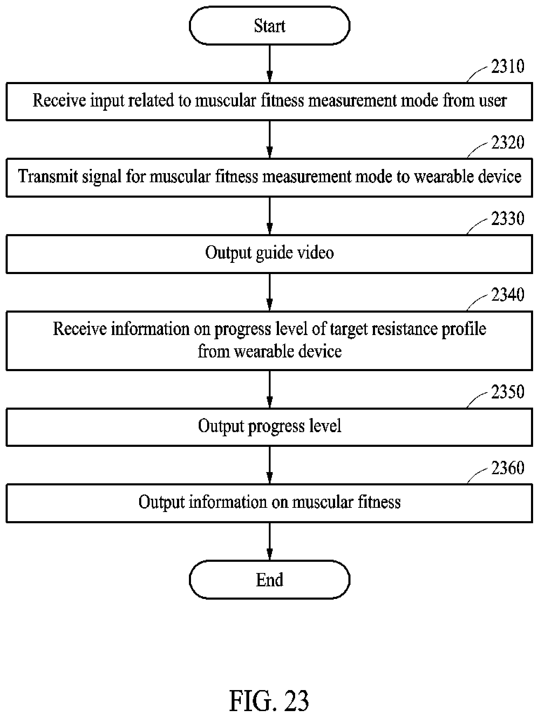

FIG. 23 is a flowchart illustrating a method of measuring muscular fitness performed by a user terminal according to an example embodiment.

According to an example embodiment, operations 2310 through 2360 to be described hereinafter with reference to FIG. 23 may be performed by a user terminal (e.g., the electronic device 200 of FIG. 2 or the user terminal 900 of FIG. 9). The user terminal may be, for example, a dedicated terminal for controlling the wearable device 100 or a terminal in which an application for controlling the wearable device 100 is installed.

In operation 2310, the user terminal may receive an input associated with a muscular fitness measurement mode from a user. For example, the user may select the muscular fitness measurement mode from among a plurality of operation modes of the wearable device 100 through the user terminal.

In operation 2320, the user terminal may transmit a signal for the muscular fitness measurement mode to the wearable device 100. For example, the user terminal and the wearable device 100 may be connected through a wireless network.

In operation 2330, the user terminal may output a target movement guide video. The guide video may be about one or more movements to be performed by the user to measure muscular fitness.

In operation 2340, the user terminal may receive information on a progress level of a target resistance profile from the wearable device 100.

After the guide video is output, measurement of the muscular fitness may be started by the wearable device 100. For example, the wearable device 100 may notify the user of the start of the measurement of the muscular fitness through a visual, auditory, or tactile output.

While the user is measuring the muscular fitness through the wearable device 100, the user terminal may receive information on an ongoing muscular fitness measurement session from the wearable device 100.

In operation 2350, the user terminal may output the received information on the progress level of the target resistance profile. For example, the user terminal may output the information on the muscular fitness measurement session (refer to FIG. 16).

In operation 2360, the user terminal may output information on the muscular fitness. For example, the information on the muscular fitness may be quantified to be numerically represented.

According to an example embodiment, the user terminal may receive measured state information from the wearable device 100 and calculate the muscular fitness based on the state information. For example, operations 1810 through 1858 described above with reference to FIG. 18 may be performed by a processor of the user terminal to calculate the muscular fitness.

According to another example embodiment, the user terminal may receive information on calculated muscular fitness from the wearable device 100 and output the received information on the muscular fitness.

The units described herein may be implemented using hardware components and software components. For example, the hardware components may include microphones, amplifiers, band-pass filters, audio to digital convertors, non-transitory computer memory and processing devices. A processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such as parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

Example embodiments include non-transitory computer-readable media including program instructions to implement various operations embodied by a computer. The media may also include, alone or in combination with the program instructions, data files, data structures, tables, and the like. The media and program instructions may be those specially designed and constructed for the purposes of example embodiments, or they may be of the kind well known and available to those having skill in the computer software arts. Examples of non-transitory computer-readable media include magnetic media such as hard disks, floppy disks, and magnetic tape; optical media such as CD ROM disks; magneto-optical media such as floptical disks; and hardware devices that are specially configured to store and perform program instructions, such as read-only memory devices (ROM) and random-access memory (RAM). Examples of program instructions include both machine code, such as produced by a compiler, and files containing higher level code that may be executed by the computer using an interpreter.

The described hardware devices may be configured to act as one or more software modules in order to perform the operations of the above-described example embodiments, or vice versa.

While this disclosure includes specific examples, it will be apparent after an understanding of the disclosure of this application that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents.

Therefore, the scope of the disclosure is defined not by the detailed description, but by the claims and their equivalents, and all variations within the scope of the claims and their equivalents are to be construed as being included in the disclosure.

**Claims**

**1.** A method of measuring muscular fitness performed by a wearable device, the method comprising:

determining a target resistance profile for a target movement to be performed by a user wearing the wearable device;

controlling a motor driver circuit of the wearable device based on the target resistance profile to control a motor of the wearable device in providing a resistance force to the user;

obtaining state information of a movement performed by the user under the resistance force; and

determining muscular fitness of the user based on the state information.

2. The method of claim 1, further comprising:

transmitting information of the determined muscular fitness to a user terminal.

3. The method of claim 1, wherein the determining of the target resistance profile comprises:

determining the target resistance profile from among a plurality of target resistance profiles, based on a resistance level received from a user terminal.

4. The method of claim 1, wherein the controlling of the motor driver circuit comprises:

determining a ratio of a time for which the motor driver circuit is controlled to be a closed loop electrically connected to the motor of the wearable device to a time for which the motor driver circuit is controlled to be an open loop, based on the target resistance profile; and

controlling the motor through the motor driver circuit, based on the determined ratio.

5. The method of claim 4, wherein the motor driver circuit comprises at least one switch, the switch being controlled based on the determined ratio.

6. The method of claim 4, wherein the ratio is represented by pulse-width modulation (PWM).

7. The method of claim 6, wherein the resistance force to be provided to the user is adjusted by the ratio of the time for which the motor driver circuit is controlled to be the closed loop electrically connected to the motor to the time for which the motor driver circuit is controlled to be the open loop within a repetitive time of the PWM,

wherein, based on the ratio of the time for which the motor driver circuit is controlled to be the closed loop increases, the resistance force increases.

8. The method of claim 1, wherein the measuring of the muscular fitness of the user based on the state information comprises:

calculating a plurality of preset indicators based on the state information; and

calculating the muscular fitness based on the plurality of preset indicators.

9. The method of claim 8, wherein the plurality of preset indicators comprise at least one of peak torque, work, muscular power, muscular endurance, torque acceleration energy, acceleration time, and a range of motion.

10. The method of claim 1, further comprising:

transmitting a progress level of the target resistance profile to the user terminal.

11. The method of claim 1, further comprising:

receiving, from a user terminal, a signal associated with a muscular fitness measurement mode as an operation mode for controlling the wearable device.

12. The method of claim 1, further comprising:

receiving, from the user, an input that selects an assistance mode as an operation mode for controlling the wearable device;

based on receiving the input that selects the assistance mode, calculating an assistance torque value for a first j oint of the user based on a first angle of the first j oint measured using a sensor; and

providing an assistance force to the user by controlling the motor driver circuit based on the assistance torque value.

13. A non-transitory computer-readable storage medium storing instructions that are executable by a processor to perform the method of claim 1.

14. A wearable device, comprising:

a memory in which a program for measuring muscular fitness of a user is recorded;
a processor configured to execute the program;
a communication module configured to exchange data with an external device;
a sensor configured to measure a first angle of a first joint of the user;
a motor driver circuit configured to be controlled by the processor; and
a motor electrically connected to the motor driver circuit,
wherein the program is configured to perform the following operations of

determining a target resistance profile for a target movement to be performed by the user wearing the wearable device;
controlling the motor driver circuit of the wearable device based on the target resistance profile to control the motor in providing a resistance force to the user;
obtaining state information of a movement performed by the user under the resistance fore; and
determining muscular fitness of the user based on the state information.

15. An exoskeleton for measuring muscular fitness of a limb of a user, comprising:

a first support member configured to be put on a first limb part of the limb of a user;
a second support member configured to be put on a second limb part of the limb of the user;
a driver and sensor, the driver and the sensor being connected to at least one of the first and the second support member;
a memory storing a program for measuring muscular fitness;
a processor configured to execute the program, the program being configured to:

determining a target resistance profile for a target movement to be performed by the user wearing the exoskeleton on the limb;
control the driver to apply the target resistance profile;
obtaining information regarding the movement of the first and the second support members;
determining the muscular fitness of the first limb part and the second limb parts of the user,

wherein the muscular fitness relates to at least one of peak torque, work, muscular power, muscular endurance, torque acceleration energy, acceleration time, and a range of motion.

FIG. 1A

FIG. 1B

100

| Memory 144 | Input interface 146 | Battery 150 |

Sensor 121

Processor 142

Motor driver circuit 112

Motor 114

Communication module 152

FIG. 1C

100-1

```
                          ┌──────────┐
                          │ Battery  │
                          │   150    │
                          └──────────┘
          ┌────────────────────┬────────────────┬─────────────────┐
          │                    │                │                 │
          │          ┌─────────┴──┐      ┌───────┴────────┐        │
          │          │  Memory    │      │ Input interface│        │
          │          │   144      │      │     146        │        │
          │          └────────────┘      └────────────────┘        │
          │                │                    │                  │
          │          ┌─────┴────────────────────┴──────┐           │
   ┌──────┴──────┐   │                                 │    ┌──────┴────────┐
   │Motor driver │   │          Processor              │    │ Motor driver  │
   │circuit  112 ├───┤            142                  ├────┤ circuit 112-1 │
   └──────┬──────┘   │                                 │    └──────┬────────┘
          │          └─────────────────┬───────────────┘           │
   ┌──────┴──────┐                     │                    ┌──────┴────────┐
   │   Motor     │                     │                    │    Motor      │
   │   114       │                     │                    │    114-1      │
   └─────────────┘                     │                    └───────────────┘
   ┌─────────────┐                     │                    ┌───────────────┐
   │   Sensor    ├─────────────────────┤                    │    Sensor     │
   │   121       │                     │                    │    121-1      │
   └─────────────┘            ┌────────┴─────────┐          └───────────────┘
                             │Communication module│
                             │       152          │
                             └────────────────────┘
```

FIG. 1D

FIG. 2

FIG. 3

FIG. 4

FIG. 5

```
                          ┌──────────────┐
                          │    Start     │
                          └──────┬───────┘
                                 │
                                 ▼                        ┌─ 610
    ┌────────────────────────────────────────────────────────┐
    │        Activate muscular fitness measurement mode        │
    └────────────────────────┬───────────────────────────────┘
                             │
                             ▼                            ┌─ 620
    ┌────────────────────────────────────────────────────────┐
    │      Determine target resistance profile for target movement │
    └────────────────────────┬───────────────────────────────┘
                             │
                             ▼                            ┌─ 630
    ┌────────────────────────────────────────────────────────┐
    │     Control motor driver circuit based on target resistance profile │
    └────────────────────────┬───────────────────────────────┘
                             │
                             ▼                            ┌─ 640
    ┌────────────────────────────────────────────────────────┐
    │       Measure state information of actual movement of user │
    └────────────────────────┬───────────────────────────────┘
                             │
                             ▼                            ┌─ 650
    ┌────────────────────────────────────────────────────────┐
    │    Calculate muscular fitness of user based on state information │
    └────────────────────────┬───────────────────────────────┘
                             │
                             ▼                            ┌─ 660
    ┌────────────────────────────────────────────────────────┐
    │          Output information on muscular fitness          │
    └────────────────────────┬───────────────────────────────┘
                             │
                             ▼
                    ┌──────────────┐
                    │     End      │
                    └──────────────┘
```

# FIG. 6

Start

710

Receive command for activating muscular fitness measurement mode

To 610

# FIG. 7

From 610

810

Transmit target movement guide information to user terminal

To 620

# FIG. 8

FIG. 9

FIG. 10

From 620

↓

/ 630

/ 1110

Determine ratio between time for which motor driver circuit is controlled to be closed loop and time for which motor driver circuit is controlled to be open loop based on target resistance profile

↓

/ 1120

Control motor through motor driver circuit based on determined ratio

↓

To 640

# FIG. 11

FIG. 12

Battery
150

Processor
142

Control

Motor driver circuit
112

Power

Motor
114

FIG. 13

Processor
142

Control signal 1    Control signal 2
1                   2

Duty

High

Low

Period

First control state

First switch          Second switch
1210                  1220

Motor 114

Control
signal 1
(High)

Control
signal 2
(High)

Third switch          Fourth switch
1230                  1240

Second control state

First switch          Second switch
1210                  1220

Motor 114

Control
signal 1
(Low)

Control
signal 2
(Low)

Third switch          Fourth switch
1230                  1240

FIG. 14

Battery
150

Processor
142

Control

Motor driver circuit
112

Motor
114

FIG. 15

900

1620

1610

12:39

:20 DB ALT. CURL
12 REPS × 17.5 LBS

1630

FIG. 16

From 640

650

1710

Calculate indicators based on state information

1720

Calculate muscular fitness based on indicators

To 660

FIG. 17

From 640

```
                                                          460

        ┌────────────────────── 1810 ──────────────────────┐
        │   Determine number of repetitions during actual movement   │
        └────────────────────────────────────────────────────────────┘

  ┌─ 1820 ─┐   ┌──── 1830 ────┐   ┌─── 1840 ───┐   ┌──── 1850 ────┐
  │Determine│  │Determine maximum│ │Calculate RoM for│ │Determine torque│
  │   ME    │  │torque for each  │ │each repetition  │ │production region│
  │         │  │repetition and   │ │                 │ │for each         │
  │         │  │time stamp of    │ │                 │ │repetition       │
  │         │  │maximum torque   │ │                 │ │                 │
  └─────────┘  └─────────────────┘ └─────────────────┘ └─────────────────┘

          ┌─ 1832 ─┐  ┌─ 1834 ─┐   ┌──── 1842 ────┐  ┌─ 1852 ─┐  ┌──── 1854 ────┐
          │Determine│ │Determine│  │Determine maximum│ │Determine│ │Calculate partial│
          │peak     │ │accelera-│  │RoM among candi- │ │maximum  │ │work for each    │
          │torque   │ │tion time│  │date RoMs        │ │TAE      │ │repetition       │
          └─────────┘ └─────────┘  └─────────────────┘ └─────────┘ └─────────────────┘

                                                                   ┌─ 1856 ─┐
                                                                   │Calculate│
                                                                   │work     │
                                                                   └─────────┘

                                                                   ┌─ 1858 ─┐
                                                                   │Calculate│
                                                                   │MP       │
                                                                   └─────────┘
```

To 660

FIG. 18

Joint Angle L

ROM (peak jnt): 27.61 deg

1910

Joint Torque L (0 if trq <0)

peak torque: 2.23 Nm

1920

TotalWork: 8.95 Nm

Muscle Power: 0.65 W

TAE: 0.10 Nm

Acc. Time: 0.95 sec

FIG. 19

Start

Receive signal related to operation mode for
controlling wearable device from user
— 2010

Determine operation mode — 2015

Muscular fitness
measurement mode
To 610

Assistance mode

Measure angle of joint of user using sensor — 2020

Calculate assistance torque value for
joint based on angle
— 2030

Provide assistance force to user by controlling
motor driver circuit based on assistance torque value
— 2040

End

FIG. 20

From 2020

2030

Is muscular fitness of user measured? 2110

No

Yes

Adjust gain and delay for state factor based on measured muscular fitness of user 2120

Calculate assistance torque value based on state factor 2130

To 2040

FIG. 21

FIG. 22A

FIG. 22B

FIG. 22C

```
            ┌─────────────┐
            │    Start    │
            └─────────────┘
                   │
                   ▼                              ╱─2310
┌────────────────────────────────────────────────────────┐
│ Receive input related to muscular fitness measurement mode from user │
└────────────────────────────────────────────────────────┘
                   │
                   ▼                              ╱─2320
┌────────────────────────────────────────────────────────┐
│ Transmit signal for muscular fitness measurement mode to wearable device │
└────────────────────────────────────────────────────────┘
                   │
                   ▼                              ╱─2330
┌────────────────────────────────────────────────────────┐
│                   Output guide video                   │
└────────────────────────────────────────────────────────┘
                   │
                   ▼                              ╱─2340
┌────────────────────────────────────────────────────────┐
│  Receive information on progress level of target resistance profile │
│                   from wearable device                 │
└────────────────────────────────────────────────────────┘
                   │
                   ▼                              ╱─2350
┌────────────────────────────────────────────────────────┐
│                  Output progress level                 │
└────────────────────────────────────────────────────────┘
                   │
                   ▼                              ╱─2360
┌────────────────────────────────────────────────────────┐
│           Output information on muscular fitness       │
└────────────────────────────────────────────────────────┘
                   │
                   ▼
            ┌─────────────┐
            │     End     │
            └─────────────┘
```

FIG. 23

| INTERNATIONAL SEARCH REPORT | International application No. |
|---|---|
| | **PCT/KR2022/005110** |

**A. CLASSIFICATION OF SUBJECT MATTER**

**B25J 9/00**(2006.01)i; **B25J 19/02**(2006.01)i; **B25J 13/08**(2006.01)i; **G01L 1/14**(2006.01)i

According to International Patent Classification (IPC) or to both national classification and IPC

**B. FIELDS SEARCHED**

Minimum documentation searched (classification system followed by classification symbols)

B25J 9/00(2006.01); A61B 5/02(2006.01); A61F 5/01(2006.01); A61H 1/00(2006.01); A61H 3/00(2006.01); A63B 24/00(2006.01); A63B 71/06(2006.01); B25J 11/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 외골격(exoskeleton), 웨어러블(wearable), 근체력(muscular fitness), 저항력(resistance), 토크(torque), 가속(acceleration)

**C. DOCUMENTS CONSIDERED TO BE RELEVANT**

| Category* | Citation of document, with indication, where appropriate, of the relevant passages | Relevant to claim No. |
|---|---|---|
| X | US 2020-0060921 A1 (PARKER-HANNIFIN CORPORATION) 27 February 2020 (2020-02-27)<br>See paragraphs [0036]-[0038], [0055]-[0059] and [0064]-[0068], claims 15 and 49 and figures 1-6 and 14. | 1-2,8-9,11,13-15 |
| Y | | 3,10,12 |
| A | | 4-7 |
| Y | KR 10-2017-0083829 A (HANWHA TECHWIN CO., LTD.) 19 July 2017 (2017-07-19)<br>See paragraphs [0028]-[0030], [0036], [0056]-[0060], [0066], [0072]-[0074], [0088] and [0089] and figure 2. | 3,10,12 |
| A | KR 10-2010-0122692 A (SAMSUNG LIFE PUBLIC WELFARE FOUNDATION) 23 November 2010 (2010-11-23)<br>See paragraphs [0041]-[0043] and figure 1. | 1-15 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 July 2022** | **08 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/005110**

**C.    DOCUMENTS CONSIDERED TO BE RELEVANT**

| Category* | Citation of document, with indication, where appropriate, of the relevant passages | Relevant to claim No. |
|---|---|---|
| A | KR 10-2012-0057081 A (BIONICS CO., LTD.) 05 June 2012 (2012-06-05)<br>See paragraphs [0092]-[0095] and figures 1, 3 and 7. | 1-15 |
| A | KR 10-2020-0061611 A (SANGMYUNG UNIVERSITY CHEONAN COUNCIL FOR INDUSTRY-ACADEMIC COOPERATION FOUNDATION) 03 June 2020 (2020-06-03)<br>See paragraphs [0045] and [0047] and figure 3. | 1-15 |

Form PCT/ISA/210 (second sheet) (July 2019)

**INTERNATIONAL SEARCH REPORT**
**Information on patent family members**

International application No.

**PCT/KR2022/005110**

| Patent document cited in search report | | | Publication date (day/month/year) | Patent family member(s) | | | Publication date (day/month/year) |
|---|---|---|---|---|---|---|---|
| US | 2020-0060921 | A1 | 27 February 2020 | EP | 3523809 | A1 | 14 August 2019 |
| | | | | WO | 2018-175004 | A1 | 27 September 2018 |
| KR | 10-2017-0083829 | A | 19 July 2017 | | None | | |
| KR | 10-2010-0122692 | A | 23 November 2010 | KR | 10-1033373 | B1 | 09 May 2011 |
| KR | 10-2012-0057081 | A | 05 June 2012 | KR | 10-1221046 | B1 | 13 February 2013 |
| KR | 10-2020-0061611 | A | 03 June 2020 | | None | | |

Form PCT/ISA/210 (patent family annex) (July 2019)